(19) Europäisches Patentamt
European Patent Office
Office européen des brevets

(11) EP 1 220 857 B1

(12) EUROPEAN PATENT SPECIFICATION

(45) Date of publication and mention of the grant of the patent: **21.09.2005 Bulletin 2005/38**

(21) Application number: **00973591.1**

(22) Date of filing: **16.10.2000**

(51) Int Cl.[7]: **C07D 487/04**, A61K 31/505, A61P 5/02

(86) International application number: **PCT/US2000/028677**

(87) International publication number: **WO 2001/029044 (26.04.2001 Gazette 2001/17)**

# (54) GONADOTROPIN RELEASING HORMONE RECEPTOR ANTAGONISTS AND THEIR RELATED METHODS OF USE

GONADOTROPIN FREISETZENDEN HORMON REZEPTOR ANTAGONISTEN UND IHRE VERWANDTEN VERWENDUNGEN

ANTAGONISTES DU RECEPTEUR DE L'HORMONE LIBERANT DE LA GONADOTROPINE ET METHODES D'UTILISATION

(84) Designated Contracting States:
**AT BE CH CY DE DK ES FI FR GB GR IE IT LI LU MC NL**

(30) Priority: **15.10.1999 US 418768**

(43) Date of publication of application: **10.07.2002 Bulletin 2002/28**

(73) Proprietor: **NEUROCRINE BIOSCIENCES, INC.
San Diego, CA 92130 (US)**

(72) Inventors:
- **ZHU, Yun-Fei
San Diego, CA 92129 (US)**
- **GROSS, Timothy, D.
San Diego, CA 92124 (US)**
- **GAO, Yinghong
San Diego, CA 92122 (US)**
- **CONNORS, Patrick, J., Jr.
San Diego, CA 92126 (US)**
- **GUO, Zhiqiang
San Diego, CA 92126 (US)**
- **CHEN, Chen
San Diego, CA 92129 (US)**

(74) Representative: **Grünecker, Kinkeldey, Stockmair & Schwanhäusser Anwaltssozietät
Maximilianstrasse 58
80538 München (DE)**

(56) References cited:
**WO-A-99/33831** **WO-A-99/44339**
**WO-A-99/51232**

- **N. CHO ET. AL.: "Discovery of a Novel, Potent and Orally Active Nonpeptide Antagonist of the Human Luteinizing Hormone Releasing Hormone (LHRH) Receptor" JOURNAL OF MEDICINAL CHEMISTRY, vol. 41, no. 22, 22 October 1998 (1998-10-22), pages 4190-5, XP002156492**
- **S. C. KOERBER ET. AL.: "Consensus Bioactive Conformation of Cyclic GnRH Antagonists Defined by NMR and Molecular Modelling." JOURNAL OF MEDICNAL CHEMISTRY, vol. 43, no. 5, 9 March 2000 (2000-03-09), pages 819-28, XP002156493**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 220 857 B1

## Description

TECHNICAL FIELD

This invention relates generally to gonadotropin-releasing hormone (GnRH) receptor antagonists, and to methods of treating disorders by administration of such antagonists to a warm-blooded animal in need thereof.

BACKGROUND OF THE INVENTION

Gonadotropin-releasing hormone (GnRH), also known as luteinizing hormone-releasing hormone (LHRH), is a decapeptide (pGlu-His-Trp-Ser-Tyr-Gly-Leu-Arg-Pro-Gly-$NH_2$) that plays an important role in human reproduction. GnRH is released from the hypothalamus and acts on the pituitary gland to stimulate the biosynthesis and release of luteinizing hormone (LH) and follicle-stimulating hormone (FSH). LH released from the pituitary gland is responsible for the regulation of gonadal steroid production in both males and females, while FSH regulates spermatogenesis in males and follicular development in females.

Due to its biological importance, synthetic antagonists and agonists to GnRH have been the focus of considerable attention, particularly in the context of prostate cancer, breast cancer, endometriosis, uterine leiomyoma, and precocious puberty. For example, peptidic GnRH agonists, such as leuprorelin (pGlu-His-Trp-Ser-Tyr-D-Leu-Leu-Arg-Pro-NHEt), have been used to treat such conditions. Such agonists appear to function by binding to the GnRH receptor in the pituitary gonadotropins, thereby inducing the synthesis and release of gonadotropins. Chronic administration of GnRH agonists depletes gonadotropins and subsequently down-regulates the receptor, resulting in suppression of steroidal hormones after some period of time (*e.g.*, on the order of 2-3 weeks following initiation of chronic administration).

In contrast, GnRH antagonists are believed to suppress gonadotropins from the onset, and thus have received the most attention over the past two decades. To date, some of the primary obstacles to the clinical use of such antagonists have been their relatively low bioavailability and adverse side effects caused by histamine release. However, several peptidic antagonists with low histamine release properties have been reported, although they still must be delivered via sustained delivery routes (such as subcutaneous injection or intranasal spray) due to limited bioavailability.

In view of the limitations associated with peptidic GnRH antagonists, a number of nonpeptidic compounds have been proposed. For example, Cho et al. (*J. Med. Chem. 41*:4190-4195, 1998) discloses thieno[2,3-*b*]pyridin-4-ones for use as GnRH receptor antagonists; U.S. Patent Nos. 5,780,437 and 5,849,764 teach substituted indoles as GnRH receptor antagonists (as do published PCTs WO 97/21704, 98/55479, 98/55470, 98/55116, 98/55119, 97/21707, 97/21703 and 97/21435); published PCT WO 96/38438 discloses tricyclic diazepines as GnRH receptor antagonists; published PCTs WO97/14682, 97/14697 and 99/09033 disclose quinoline and thienopyridine : derivatives as GnRH antagonists; published PCTs WO 97/44037, 97/44041, 97/44321 and 97/44339 teach substituted quinolin-2-ones as GnRH receptor antagonists; and published PCT WO 99/33831 discloses certain phenyl-substituted fused nitrogen-containing bicyclic compounds as GnRH receptor antagonists. PCT WO 99/51232 discloses antogonists of GnRH having a bicyclic structure. N. Cho et al. disclose in J.Med.Chem. 1998,41,4190- 4195 nonpeptide antagonists of the human luteinizing hormone-releasing hormone (LHRH) receptor.

While significant strides have been made in this field, there remains a need in the art for effective small molecule GnRH receptor antagonists. There is also a need for pharmaceutical compositions containing such GnRH receptor antagonists, as well as methods relating to the use thereof to treat, for example, sex-hormone related conditions. The present invention fulfills these needs, and provides other related advantages.

SUMMARY OF THE INVENTION

In brief, this invention is generally directed to gonadotropin-releasing hormone (GnRH) receptor antagonists, as well as to methods for their preparation and use, and to pharmaceutical compositions containing the same. More specifically, the GnRH receptor antagonists of this invention are compounds having the following general structure (I):

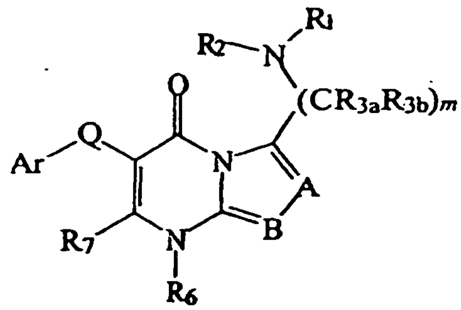

(I)

including stereoisomers, and pharmaceutically acceptable salts thereof, wherein Ar, A, B, Q, $R_1$, $R_2$, $R_{3a}$, $R_{3b}$, $R_6$, $R_7$ and *m* are as defined below.

The GnRH receptor antagonists of this invention have utility over a wide range of therapeutic applications, and may be used to treat a variety of sex-hormone related conditions in both men and women, as well as a mammal in general (also referred to herein as a "subject"). For example, such conditions include endometriosis, uterine fibroids, polycystic ovarian disease, hirsutism, precocious puberty, gonadal steroid-dependent neoplasia such as cancers of the prostate, breast and ovary, gonadotropbe pituitary adenomas, sleep apnea, irritable bowel syndrome, premenstrual syndrome, benign prostatic hypertrophy, contraception and infertility (e.g., assisted reproductive therapy such as *in vitro* fertilization). The compounds of this invention are also useful as an adjunct to treatment of growth hormone deficiency and short stature, and for the treatment of systemic lupus erythematosis. The compounds are also useful in combination with androgens, estrogens, progesterones, and antiestrogens and antiprogestogens for the treatment of endometriosis, fibroids, and in contraception, as well as in combination with an angiotensin-converting enzyme inhibitor, an angiotensin II-receptor antagonist, or a renin inhibitor for the treatment of uterine fibroids. In addition, the compounds may be used in combination with bisphosphonates and other agents for the treatment and/or prevention of disturbances of calcium, phosphate and bone metabolism, and in combination with estrogens, progesterones and/or androgens for the prevention or treatment of bone loss or hypogonadal symptoms such as hot flashes during therapy with a GnRH antagonist.

The methods of this invention include administering an effective amount of a GnRH receptor antagonist, preferably in the form of a pharmaceutical composition, to a mammal in need thereof. Thus, in still a further embodiment, pharmaceutical compositions are disclosed containing one or more GnRH receptor antagonists of this invention in combination with a pharmaceutically acceptable carrier and/or diluent.

These and other aspects of the invention will be apparent upon reference to the following detailed description. To this end, various references are set forth herein which describe in more detail certain background information, procedures, compounds and/or compositions.

## DETAILED DESCRIPTION OF THE INVENTION

As mentioned above, the present invention is directed generally to compounds useful as gonadotropin-releasing hormone (GnRH) receptor antagonists. The compounds of this invention have the following structure (I):

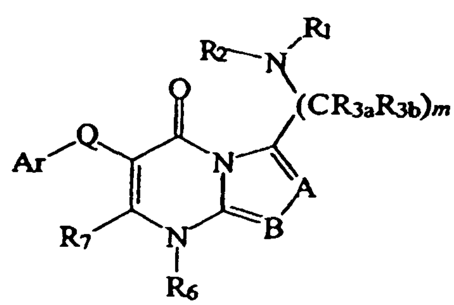

(I)

including stereoisomers, and pharmaceutically acceptable salts thereof, wherein:

A is independently selected from N or $CR_4$;
B is independently selected from N or $CR_5$;
Q is a direct bond or $-(CR_{8a}R_{8b})_r$-Z-$(CR_{10a}R_{10b})_s$-;
*m*, *r* and *s* are the same or different and selected from an integer from 0 to 6;
Z is a direct bond or -O-, -S-, $-NR_9-$, -SO-, $-SO_2-$, $-OSO_2-$, $-SO_2O-$, $-SO_2NR_9-$, $-NR_9SO_2-$, -CO-, -COO-, -OCO-, $-CONR_9-$, $-NR_9CO-$, $-NR_9CONR_{9a}$, $-OCONR_9-$ or $-NR_9COO-$;
$R_1$ is hydrogen, alkyl, substituted alkyl, aryl, substituted aryl, arylalkyl, substituted arylalkyl, beteroaryl, substituted beteroaryl, heteroarylalkyl, substituted heteroarylalkyl, $-C(R_{1a})(=NR_{1b})$, or $-C(NR_{1a}R_{1c})(=NR_{1b})$;
$R_2$ is hydrogen, alkyl or substituted alkyl;
or $R_1$ and $R_2$ taken together with the nitrogen atom to which they are attached form a heterocycle ring or a substituted heterocycle ring;
$R_{3a}$ and $R_{3b}$ are independently selected from hydrogen, alkyl, substituted alkyl, aryl, substituted aryl, arylalkyl, heterocycle, heterocyclealkyl, hydroxy, alkoxy, alkylthio, alkylamino, $CONR_{14}R_{15}$, or $-COOR_{14}$;
or $R_{3a}$ and $R_{3b}$ taken together with the carbon atom to which they are attached form a 3-6 membered homocyclic ring, substituted homocyclic ring, heterocyclic ring or substituted heterocyclic ring;
or $R_{3a}$ and $R_{3b}$ taken together form $=NR_{3c}$;
$R_4$ is hydrogen, halogen, cyano, nitro, alkyl, substituted alkyl, arylalkyl, substituted arylalkyl, heterocyclealkyl, substituted heterocyclealkyl, $-COR_{11}$, $-COOR_{11}$, $-CONR_{12}R_{13}$, $-OR_{11}$, $-OCOR_{11}$, $-OSO_2R_{11}$, $-SR_{11}$, $-SO_2R_{11}$, $-NR_{12}R_{13}$, $-NR_{11}COR_{12}$,
$-NR_{11}CONR_{12}R_{13}$, $-NR_{11}SO_2R_{12}$ or $-NR_{11}SO_2NR_{12}R_{13}$; or $R_4$ and $R_1$, together with the atoms to which they are attached, form a 5-7 member heterocyclic ring or substituted heterocyclic ring;
or $R_4$ and $R_{3a}$, together with the atoms to which they are attached, form a 5-7 membered homocyclic ring, substituted homocyclic ring, heterocyclic ring or substituted heterocyclic ring;
$R_5$ is hydrogen, halogen, lower alkyl, arylalkyl, alkoxy, alkylthio, alkylamino, cyano or nitro;
$R_6$ is hydrogen, alkyl, substituted alkyl, aryl substituted aryl, arylalkyl, substituted arylalkyl, heteroaryl, substituted heteroaryl, heteroarylalkyl or substituted heteroarylalkyl;
$R_7$ is hydrogen, halogen, cyano, alkyl, substituted alkyl, alkoxy, alkylthio, alkylsulfonyl or alkylamino;
Ar is aryl, heteroaryl, substituted aryl or substituted heteroaryl; and
$R_{1a}$, $R_{1b}$, $R_{1c}$, $R_{3c}$, $R_{8a}$, $R_{8b}$, $R_9$, $R_{9a}$, $R_{10a}$, $R_{10b}$, $R_{11}$, $R_{12}$, $R_{13}$, $R_{14}$ and $R_{15}$ are the same or different and at each occurrence independently hydrogen, acyl, alkyl, substituted alkyl, aryl, substituted aryl, arylalkyl, substituted arylalkyl, heterocycle, substituted heterocycle, heterocyclealkyl or substituted heterocyclealkyl;
or $R_{1a}$ and $R_{1b}$, $R_{8a}$ and $R_{8b}$, $R_{10a}$ and $R_{10b}$, or $R_{12}$ and $R_{13}$ taken together with the atom or atoms to which they are attached form a homocyclic ring, substituted homocyclic ring, heterocyclic ring or substituted heterocyclic ring.

As used herein, the above terms have the following meaning:

"Alkyl" means a straight chain or branched, noncyclic or cyclic, unsaturated or saturated aliphatic hydrocarbon containing from 1 to 10 carbon atoms, while the term "lower alkyl" has the same meaning as alkyl but contains from 1 to 6 carbon atoms. Representative saturated straight chain alkyls include methyl, ethyl, n-propyl, n-butyl, n-pentyl, n-hexyl, while saturated branched alkyls include isopropyl, *sec*-butyl, isobutyl, *tert*-butyl, isopentyl. Representative saturated cyclic alkyls include cyclopropyl, cyclobutyl, cyclopentyl, cyclohexyl, while unsaturated cyclic alkyls include cyclopentenyl and cyclohexenyl, Cyclic alkyls are also referred to herein as "homocyclic rings." Unsaturated alkyls contain at least one double or triple bond between adjacent carbon atoms (referred to as an "alkenyl" or "alkynyl", respectively). Representative straight chain and branched alkenyls include ethylenyl, propylenyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl,; while representative straight chain and branched alkynyls include acetylenyl, propynyl, 1-butynyl, 2-butynyl, l-pentynyl, 2-pentynyl, 3-methyl-1 butynyl.
"Aryl" means an aromatic carbocyclic moiety such as phenyl or naphthyl.
"Arylalkyl" means an alkyl having at least one alkyl hydrogen atoms replaced with an aryl moiety, such as benzyl, $-(CH_2)_2$phenyl, $-(CH_2)_3$phenyl, $-CH(phenyl)_2$,.
"Heteroaryl" means an aromatic heterocycle ring of 5- to 10 members and having at least one heteroatom selected from nitrogen, oxygen and sulfur, and containing at least I carbon atom, including both mono- and bicyclic ring systems. Representative heteroaryls are furyl, benzofuranyl, thiophenyl, benzothiophenyl, pyrrolyl, indolyl, isoindolyl, azaindolyl, pyridyl, quinolinyl, isoquinolinyl, oxazolyl, isooxazolyl, benzoxazolyl, pyrazolyl, imidazolyl, benzimidazolyl, thiazolyl, benzothiazolyl, isothiazolyl, pyridazinyl, pyrimidinyl, pyrazinyl, triazinyl, cinnolinyl, phthalazinyl, and quinazolinyl.
"Heteroarylalkyl" means an alkyl having at least one alkyl hydrogen atom replaced with a heteroaryl moiety, such as $-CH_2$pyridinyl, $-CH_2$pyrimidinyl

"Heterocycle" (also referred to herein as a "heterocycle ring") means a 5- to 7-membered monocyclic, or 7- to 10-membered bicyclic, heterocyclic ring which is either saturated, unsaturated, or aromatic, and which contains from I to 4 heteroatoms independently selected from nitrogen, oxygen and sulfur, and wherein the nitrogen and sulfur heteroatoms may be optionally oxidized, and the nitrogen heteroatom may be optionally quaternized, including bicyclic rings in which any of the above heterocycles are fused to a benzene ring. The heterocycle may be attached via any heteroatom or carbon atom. Heterocycles include heteroaryls as defined above. Thus, in addition to the heteroaryls listed above, heterocycles also include morpholinyl, pyrrolidinonyl, pyrrolidinyl, piperidinyl, hydantoinyl, valerolactamyl, oxiranyl, oxctanyl, tetrahydrofuranyl, tetrahydropyranyl, tetrahydropyridinyl, tetrahydroprimidinyl, tetrahydrothiophenyl, tetrahydrothiopyranyl, tetrahydropyrimidinyl, tetrahydrothiophenyl, tetrahydrothiopyranyl.

"Heterocyclealkyl" means an alkyl having at least one alkyl hydrogen atom replaced with a heterocycle, such as $-CH_2$morpholinyl.

"Homocycle" means a saturated or unsaturated (but not aromatic) carbocyclic ring containing from 5-7 carbon atoms, such as cyclopentane, cyclohexane, cycloheptane, cyclohexene.

The term "substituted" as used herein means any of the above groups (*i.e.*, alkyl, aryl, arylalkyl, heteroaryl, heteroarylalkyl, homocycle, heterocycle and heterocyclealkyl) wherein at least one hydrogen atom is replaced with a substituent. In the case of a keto substituent ("-C(=O)-") two hydrogen atoms are replaced. When substituted, "substituents" within the context of this invention include halogen, hydroxy, cyano, nitro, amino, alkylamino, dialkylamino, alkyl, alkoxy, alkylthio, baloalkyl, aryl, substituted aryl, arylalkyl, substituted arylalkyl, heteroaryl, substituted beteroaryl, heteroarylalkyl, substituted heteroarylalkyl, heterocycle, substituted heterocycle, heterocyclealkyl, substituted heterocyclealkyl, $-NR_aR_b$, $-NR_aC(=O)R_b$, $-NR_aC(=O)NR_aNR_b$ , $-NR_aC(=O)OR_b$ $-NR_aSO_2R_b$, $-C(=O)R_a$ $-C(=O)OR_a$, $-C(=O)NR_aR_b$, $-OC(=O)NR_aR_b$, -SH, $-SOR_a$, $-S(=O)_2R_a$, $-OS(=O)_2R_a$, $-S(=O)_2OR_a$, wherein $R_a$and $R_b$ are the same or different and independently hydrogen, alkyl, substituted alkyl, aryl, substituted aryl, arylalkyl, substituted arylalkyl, heteroaryl, substituted heteroaryl, heteroarylalkyl, substituted heteroarylalkyl, heterocycle, substituted heterocycle, heterocyclealkyl or substituted heterocyclealkyl.

"Halogen" means fluoro, chloro, bromo and iodo.

"Haloalkyl" means an alkyl having at least one hydrogen atom replaced with halogen, such as trifluoromethyl.

"Alkoxy" means an alkyl moiety attached through an oxygen bridge (*i.e.*, -O-alkyl) such as methoxy, ethoxy.

"Alkylthio" means an alkyl moiety attached through a sulfur bridge (*i.e.*, -S-alkyl) such as methylthio, ethylthio.

"Alkylsulfonyl" means an alkyl moiety attached through a sulfonyl bridge (*i.e.*, $-SO_2$-alkyl) such as methylsulfonyl, ethylsulfonyl.

"Alkylamino" and "dialkylamino" mean one or two alkyl moiety attached through a nitrogen bridge (*i.e.*, -N-alkyl) such as methylamino, ethylamino, dimethylamino, diethylamino.

In one embodiment of this invention, Q is a direct bond and representative GnRH receptor antagonists of this invention include compounds having the following structure (II):

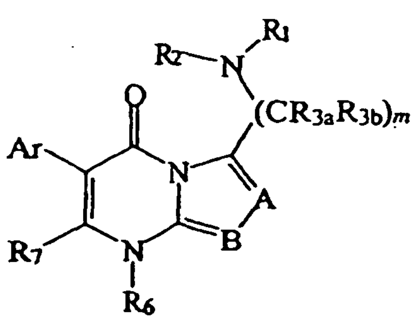

(II)

In a further embodiment of structure (II), Ar is phenyl, substituted phenyl, heteroaryl or substituted heteroaryl.

In another embodiment, Q is $-(CR_{8a}R_{8b})_r$-Z-$(CR_{10a}R_{10b})_s$-, *r* and *s* are both zero, and representative GnRH receptor antagonists of this invention include compounds having the following structure (III):

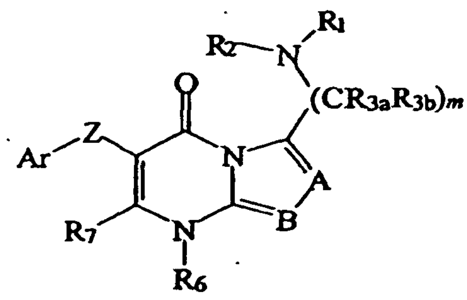

(III)

In another embodiment of structures (II), A is $CR_4$ and B is N, as represented by the following structures (IV):

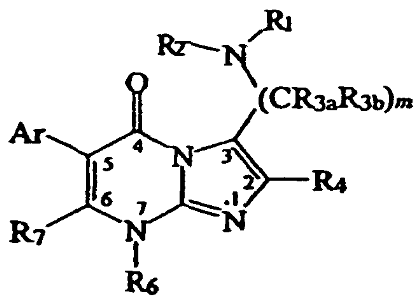

(IV)

Similarly, in another embodiment of structures (II), A is $CR_4$ and B is $CR_5$, as represented by the following structure (V):

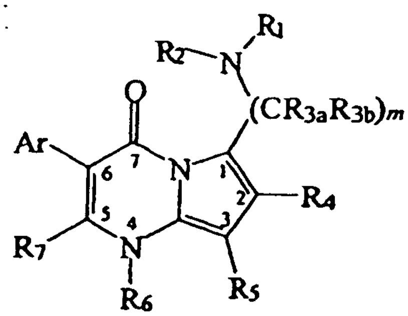

(V)

In still further embodiments of structure (II), A is N and B is $R_5$ or both A and B are N, as represented by the following structures (VI) and (VII), respectively:

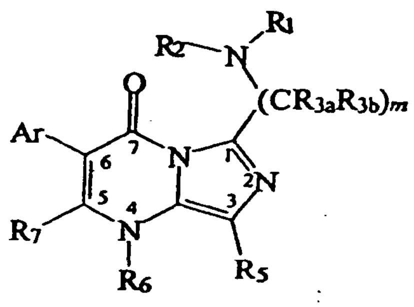

(VI)

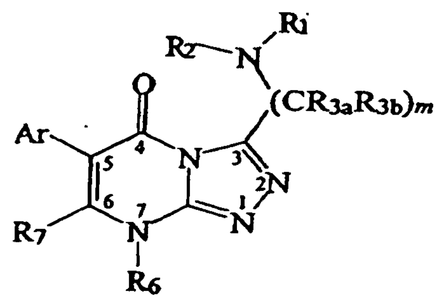

(VII)

In yet further embodiments of structure (I) of this invention, Q is a direct bond, $R_6$ is benzyl (substituted or unsubstituted), A is $CR_4$ and B is N or $CR_5$, as represented by the following structures (VIII) and (LX), respectively (wherein X represents one or more optional substituents as defined above):

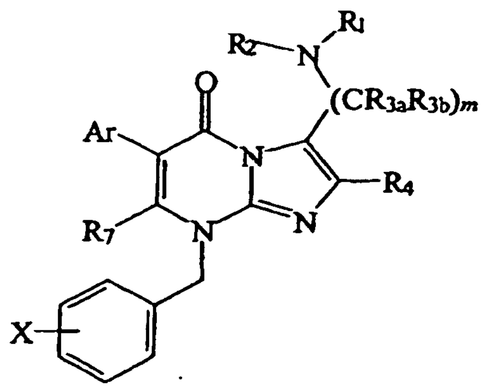

(VIII)

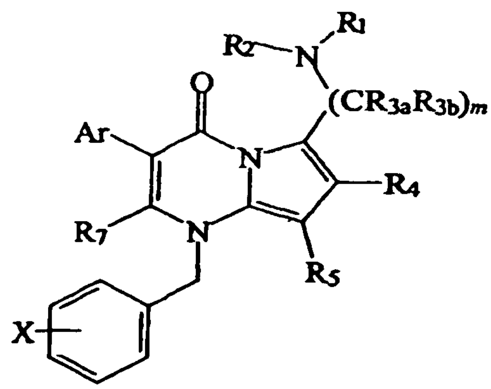

(IX)

In a more specific embodiment of structures (VIII) and (IX), *m* is 1 and both $R_{3a}$ and $R_{3b}$ are hydrogen, as represented by the following structures (X) and (XI):

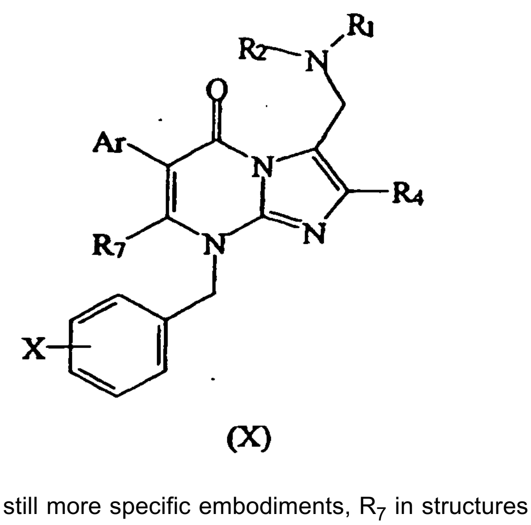

(X)

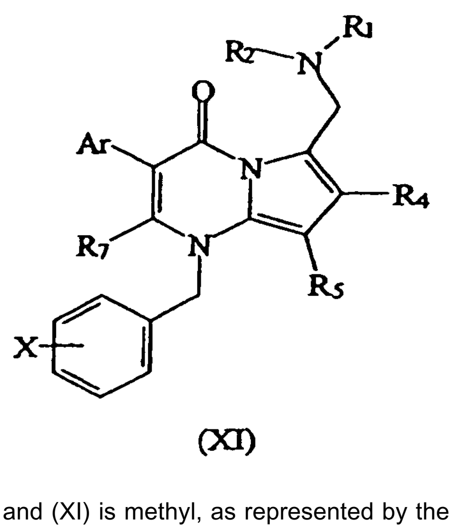

(XI)

In still more specific embodiments, $R_7$ in structures (X) and (XI) is methyl, as represented by the following structures (XII) and (XIII), respectively:

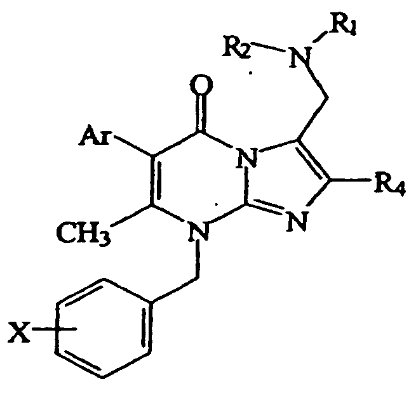

(XII)

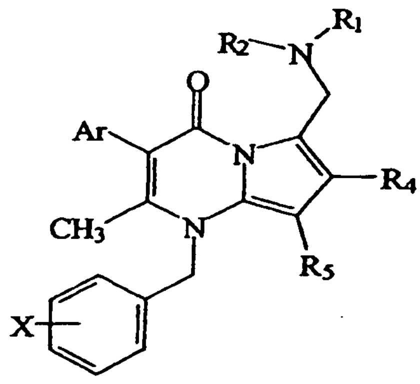

(XIII)

One class of representative compounds having structures (XII) or (XIII) include those compounds wherein Ar is aryl, substituted aryl, heteroaryl or substituted heteroaryl, such as substituted or unsubstituted phenyl, pyridyl, pyrimidinyl.

The compounds of the present invention may be prepared by known organic synthesis techniques, including the methods described in more detail in the Examples. However in general, the compounds of structure (I) above may be made by the following Reaction Schemes. Specifically, compounds wherein A is $CR_4$ and B is N may be made by Reaction Scheme A, compounds wherein A is $CR_4$ and B is $CR_5$ may be made by Reaction Scheme B, compounds wherein A is N and B is $CR_5$ may be made by Reaction Scheme C, and compounds wherein A is N and B is N may be made by Reaction Scheme D. All substituents in the following Reaction Schemes are as defined above unless indicated otherwise. To this end, $R_3$ and $R_8$ in the following Reaction Schemes are the same or different and independently hydrogen, a substituent as defined above, or the moiety -Q-Ar.

<u>Reaction Scheme A</u>

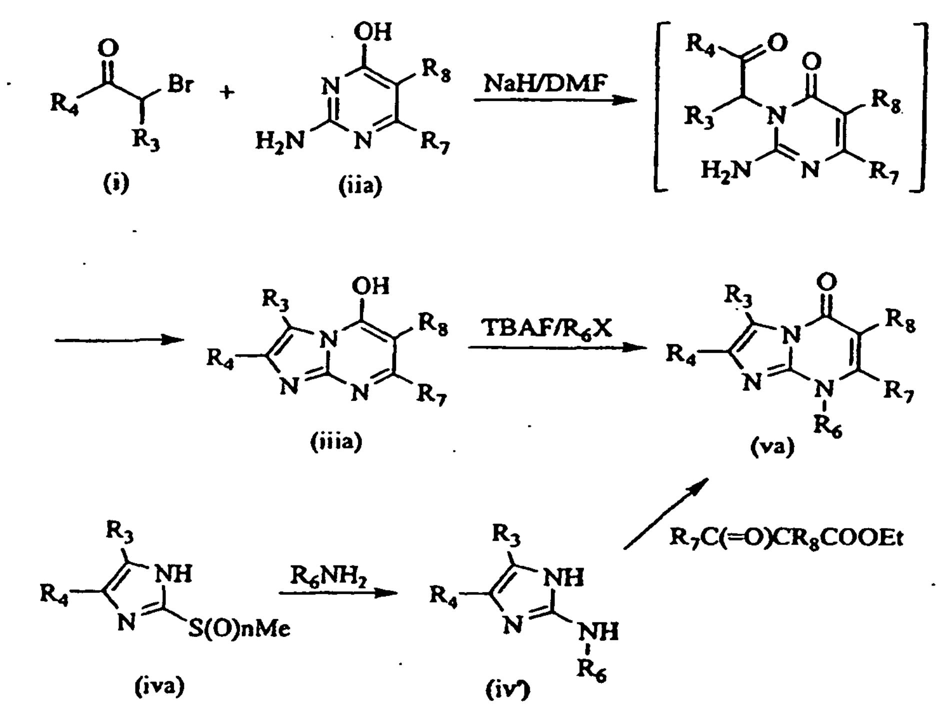


Cyclizadon of α-bromocarbonyl or α-bromocarboxylate (i) with 2-aminopyrimid-4-one (iia) in the presence of a base such as sodium hydride, tetrabutylammonium fluoride, potassium carbonate in an inert solvent such as DME, dimethylformamide, ethanol at a temperature of 25-100°C for a period of 12-24 hours gives the imidazolo[1,2-a]pyrimidone (iiia). Compound (iiia) can be modified by alkylation with an alkyl halide in the presence of a base such as TBAF, sodium hydride or silver oxide in an inert solvent such as DME, THF or DMF at a temperature of 0-100°C for a period of 1-24 hours to give the 7-alkylated imidazolo[1,2-a]pyrimid-4-one (va).

Alternatively, the imidazolopyrimidone (va) can be synthesized from imidazole (iva). Reaction of compound (iva) with a primary amine with or without a solvent such as DMF, DMSO, toluene or glycol at a temperature of 25-180°C for a period of 2-24 hours gives the 2-aminoimidazole (iv'), which reacts with a acetoacetate derivative in a solvent such as dioxane, ethanol or DMF at a temperature of 25-120°C for a period of 2-24 hours gives the desired compound (va).

**Reaction Scheme B**

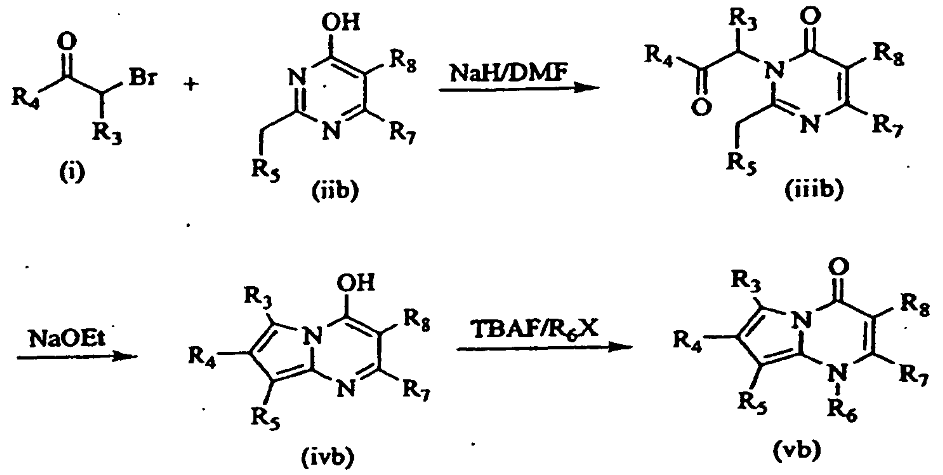


Reaction of α-bromocarbonyl (i) with 4-hydroxypyridine (iib) in the presence of a base such as sodium hydride, TBAF, or potassium carbonate in an inert solvent such as DMF, THF or ethanol at a temperature of 25-100°C for a period of 1-24 hours gives the N-alkylated pyrimidone (iiib). Compound (iiib) can be cyclized in the presence of a base such as sodium ethoxide, sodium methoxide or potassium t-butoxide in an inert solvent such as ethanol, THF or DMF at a temperature of 25-100°C for a period of 0.5 to 16 hours gives the pyrrolo[1,2-a]pyrimidone (ivb). Alkylation of compound (ivb) can be accomplished with an alkyl halide in the presence of a base such as sodium hydride, TBAF or potassium carbonate at a temperature of 25-100°C for a period of 1-24 hours give the alkylated compound (vb).

**Reaction Scheme C**

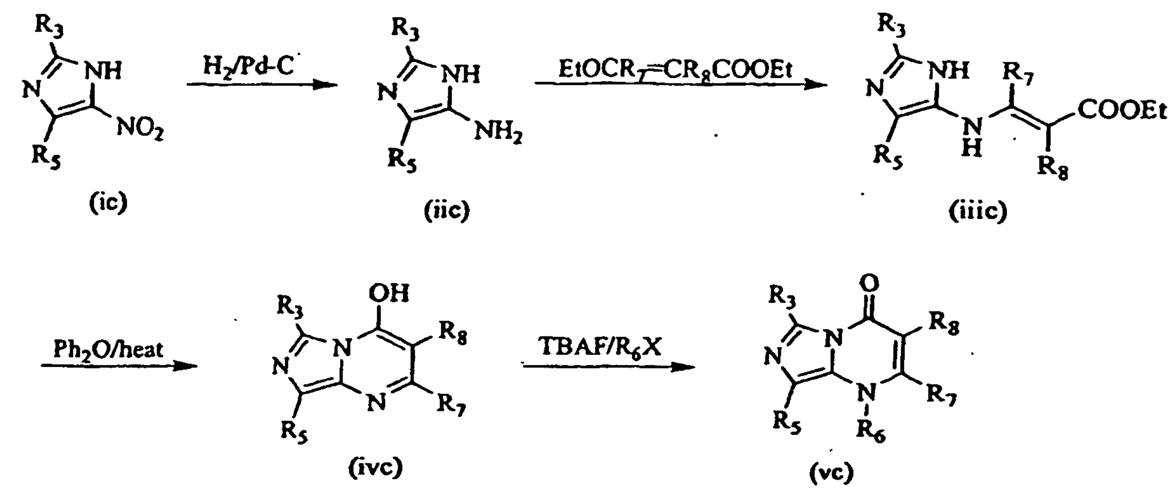


Reduction of the 4-nitroimidazole (ic) with hydrogenation catalyzed by a catalyst such as palladium on carbon,

under hydrogen atmosphere, in a solvent such as methanol, acetic acid or ethyl acetate at room temperature for a period of 1-16 hours gives the 4-aminoimidazole (iic). Reaction of compound (iic) with beta-ethoxyacrylate in a solvent such as benzene or methanol with or without a catalyst such as toluenesulfonic acid at a temperature of 25-100°C for a period of 1-16 hours gives the enamine (iiic). When the compound (iiic) is heated at a temperature of 200-280°C in a solvent such as phenyl ether for a period of 0.25 to 2 hours the cyclized product (ivc) is obtained. Compound (ivc) can be modified by alkylation in the presence of a base such as sodium hydride, TBAF or potassium carbonate in an inert solvent such as THF, DME or DMF at a temperature of 25-100°C for a period of 1-16 hours to give compound (vc).

## Reaction Scheme D

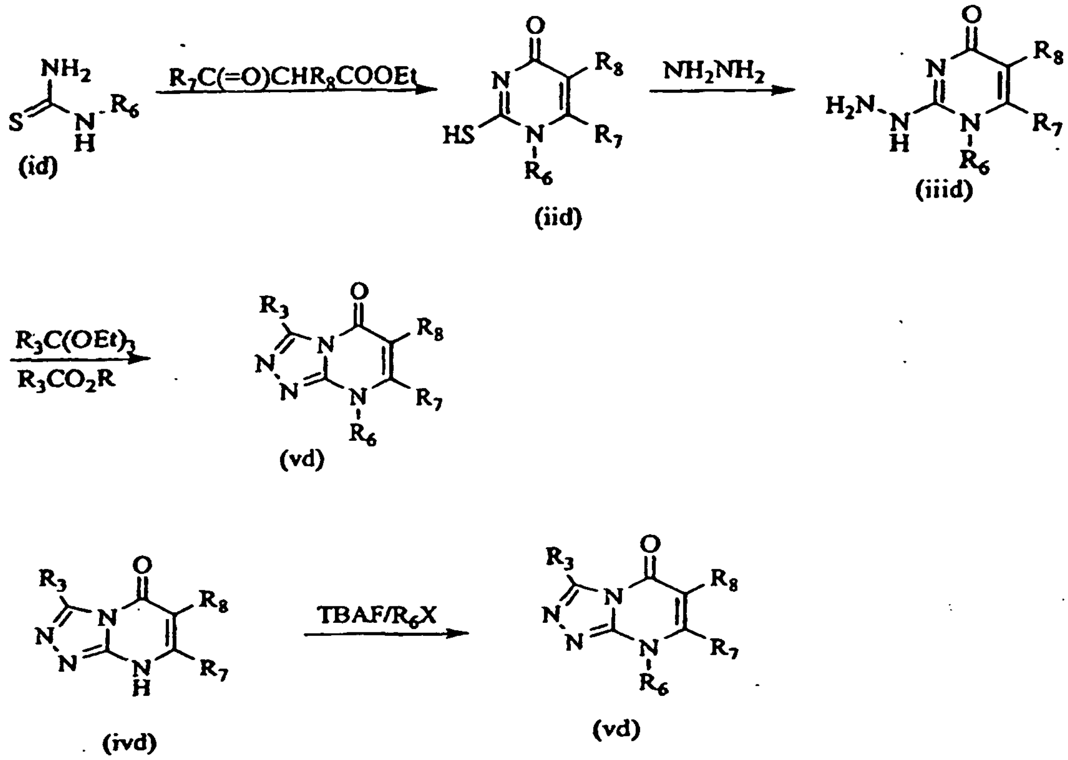


(id) (iid) (iiid) (vd) (ivd) (vd)

Cyclization of thiourea (1d) with an acetoacetate derivative in the presence of a base such as sodium methoxide or potassium t-butoxide in an inert solvent such as methanol, ethanol at a temperature of 25-120°C for a period of 2-24 hours gives the pyrimidone (iid) as one of the two isomers. Reaction of compound (iid) with hydrazine in an appropriate solvent such as ethanol or water at a temperature of 25-100°C for a period of 1-24 hours give the hydazinopyrimidine (iiid), which is cyclized upon treatment of triethoxyalkane or a carboxylic acid derivative at a temperature of 25-120°C for a period of 2-24 hours to give the 1,2,4-triazolo[1,2-a]pyrimidone (vd).

Alternatively, compound (vd) can be obtained by alkylation of compound (ivd) with an alkyl halide in the presence of a base such as sodium hydride, TBAF, potassium carbonate at a temperature of 25-100°C for a period of 1-24 hours.

## Reaction Scheme E

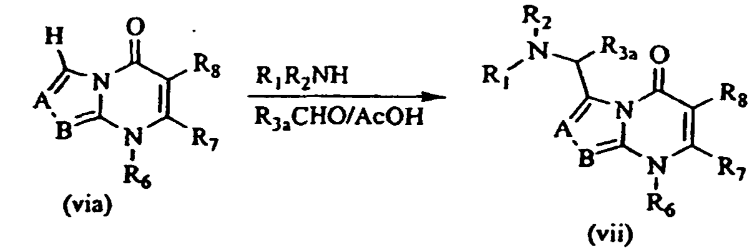


(via) (vii)

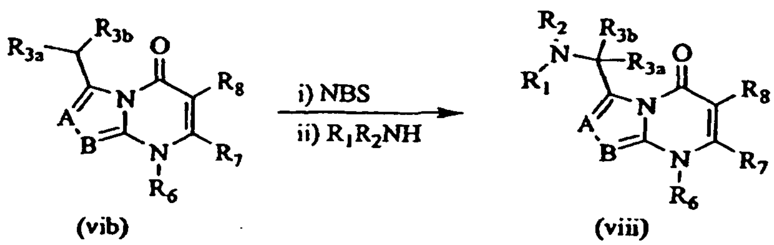


(vib) (viii)

The amino compound (vii) can be prepared by treatment of the starting material (via) with a secondary amine and an aldehyde such as formaldehyde or acetaldehyde in an appropriate solvent such as ethanol, dioxane or acetic acid at a temperature of 25-100°C for a period of 1-24 hours.

The pyrimidone (vib) can be modified by treatment with a halogenating reagent such as N-bromosuccinamide in an inert solvent such as tetrachloromethane or DMF at a temperature of 50-100°C for a period of 2-16 hours to give a halide compound, which reacts with a primary or secondary amine with or without an amine base such as triethylamine, diisopropylethylamine or pyridine in an inert solvent such as chloroform, tetrachloromethane or tetrahydrofuran at a temperature of 25-80°C for a period of 1-16 hours to give the amino analog (viii).

## Reaction Scheme F

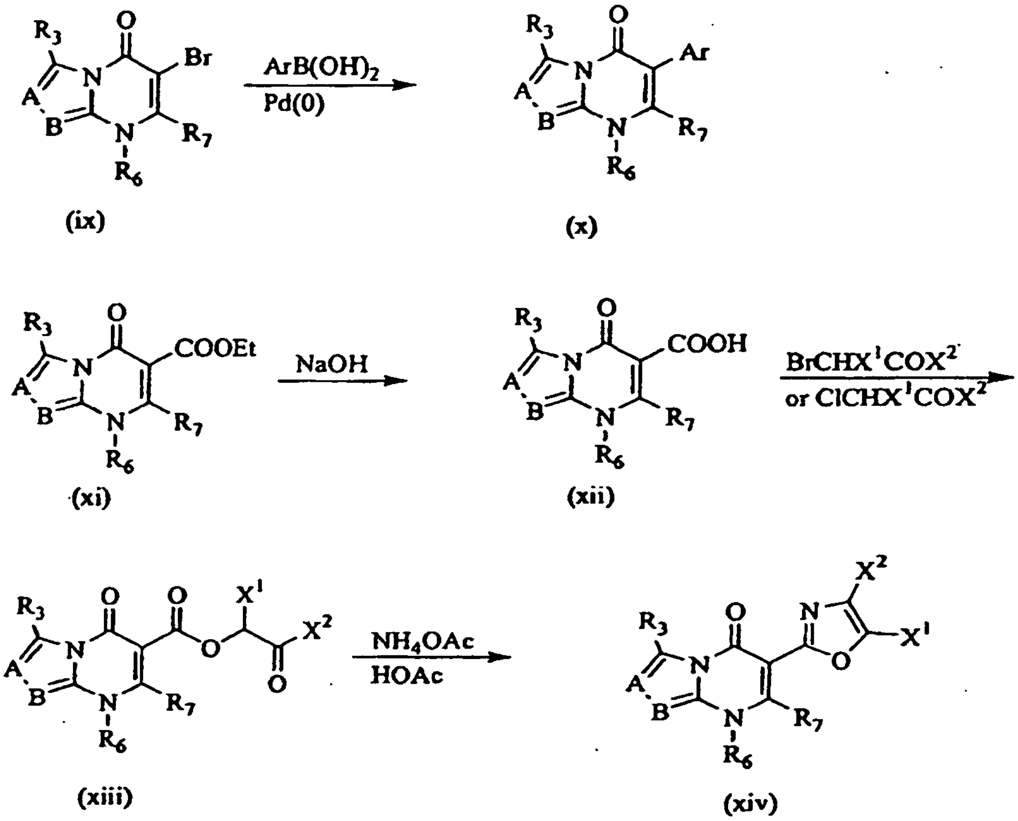


The bromopyrimidone (ix) can be converted to the corresponding aryl (Ar = phenyl or substituted phenyl) or heteroaryl (heterocyclic aromatic ring with or without substituents) compound (x) by a palladium-catalyzed cross coupling reaction of compound (ix) with an organoboronic acid in the presence of a base such as sodium carbonate, cesium fluoride, or sodium acetate in an inert solvent such as benzene, ethanol, water or mixture thereof at a temperature of 25-120°C for a period of 1-72 hours. Alternatively, heteroaryl compound such as oxazole (xiv) can be prepared by a cyclization of an ester (xiii) with an ammonium moiety such as ammonium acetate in an appropriate solvent such as acetic acid at a temperature of 25-120°C for 1-24 hours. The ester (xiii) can be synthesized from alkylation of an acid (xii) with a alpha-bromoketone in the presence of a base such as potassium carbonate or triethylamine in an inert solvent such as DMF at a temperature of 25-100°C for a period of 1-24 hours (wherein $X^1$ and $X^2$ are substituents as defined herein).

## Reaction Scheme G

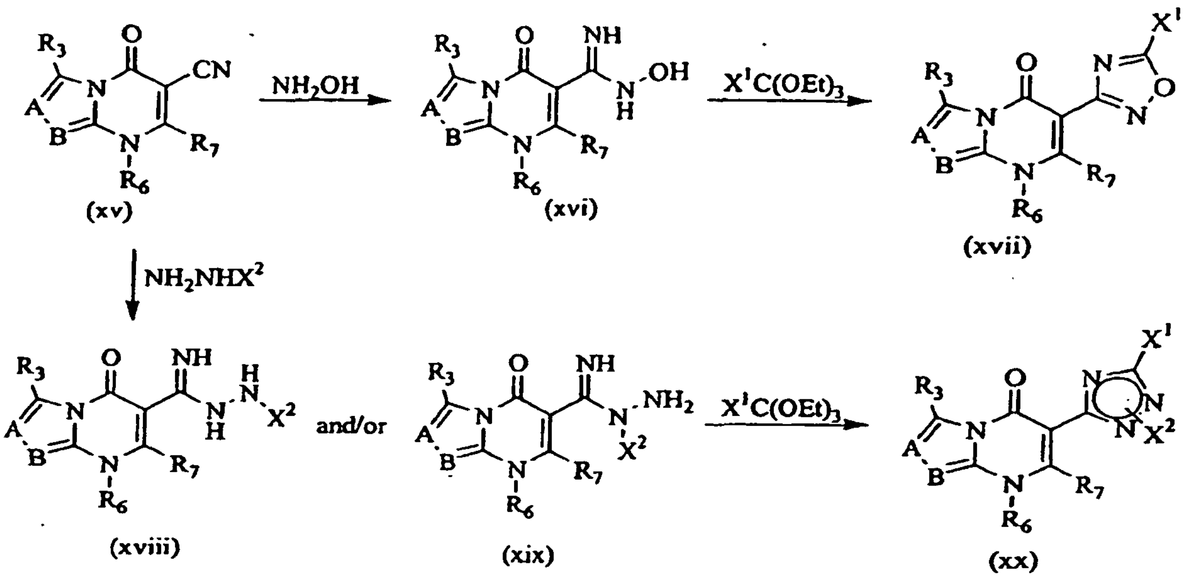


(xv) (xvi) (xvii) (xviii) (xix) (xx)

Cyano compound (xv) can be converted to the corresponding heteroaryl analogs such as oxodiazole or triazole. Reaction of compound (xv) with hydroxylamine in a solvent such as methanol, ethanol, water, dioxane or the mixture thereof at a temperature of 25-120°C for a period of 1-24 hours gives the N-hydroxylamidine (xvi), which is treated with triethoxyalkane with or without a solvent such as ethanol, dioxane or an appropriate carboxylic acid at a temperature of 25-120°C for a period of 1-24 hours to give the 1,2,3-oxodiazole (xvii) (wherein $X^1$ is a substituent as defined herein). Reaction of (xv) with hydrazine, including alkylhydrazine and arylhydrazine in an appropriate solvent such as ethanol, dioxane, water or a mixture thereof for a period of 1-24 hours gives N-aminoamidine (xviii) or (xix) (wherein $X^1$ is a substituent as defined herein), which is converted to the corresponding 1,2,3-triazole (xx) by reaction with triethoxyalkane (wherein $X^2$ is a substituent as defined herein) with or without a solvent such as dioxane, ethano, toluene, or an appropriate carboxylic acid at a temperature of 25-120°C for a period of 1-24 hours.

## Reaction Scheme H

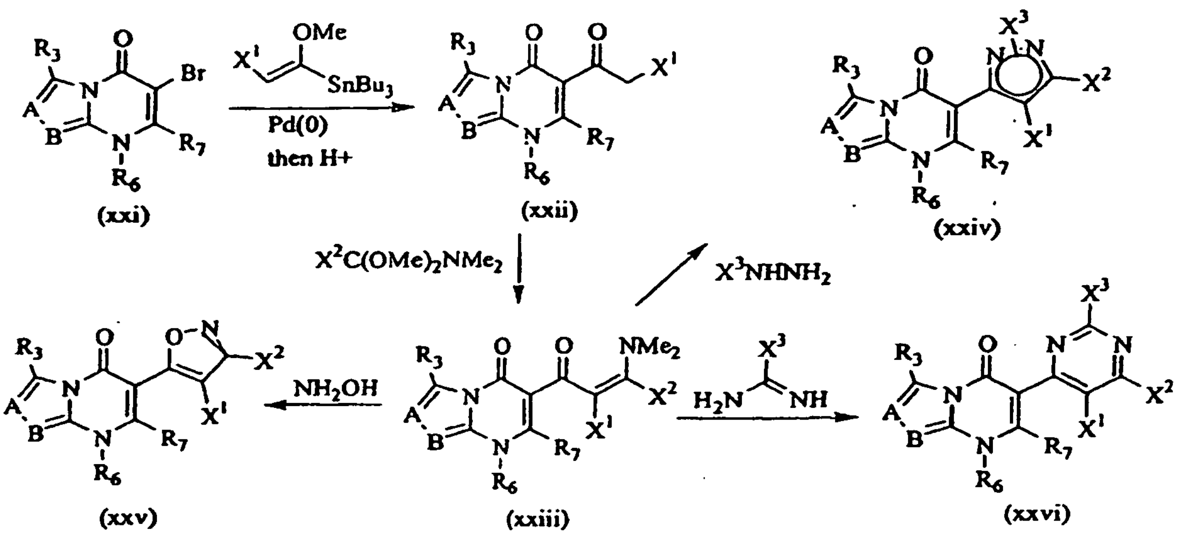


(xxi) (xxii) (xxiv) (xxv) (xxiii) (xxvi)

The bromo pyrimidone (xxi) can be modified by a palladium catalyzed cross coupling with a vinyltin in a solvent

such as THF, toluene or DMF at a temperature of 25-120°C for a period of 1-24 hours to give the ketone (xxii) (wherein $X^1$ is a substituent). Condensation of the ketone (xxii) with triethoxyalkane or dimethoxydimethylaminoalkane with or without a solvent such as toluene, dioxane or DMF at a temperature of 25-120°C for a period of 1-24 hours gives the α,β-unsaturated ketone (xxiii) (wherein $X^2$ is a substituent). Cyclization of compound (xxiii) with hydrazine including alkylhydrazine and arylhydrazine in a solvent such as ethanol, dioxane, water or a mixture thereof at a temperature of 25-120°C for a period of 1-24 hours gives pyrazole (xxiv) (wherein $X^3$ is a substituent). Cyclization of compound (xxiii) with hydroxylamine in a solvent such as ethanol, dioxane, water or a mixture thereof at a temperature of 25-120°C for a period of 1-24 hours gives isoxazole (xxv). Cyclization of compound (xxiii) with amidine, urea, thiourea or guanidine in a solvent such as ethanol, dioxane, water or a mixture thereof at a temperature of 25-120°C for a period of 1-24 hours gives pyrimidine (xxvi) (wherein $X^3$ is a substituent).

## Reaction Scheme I

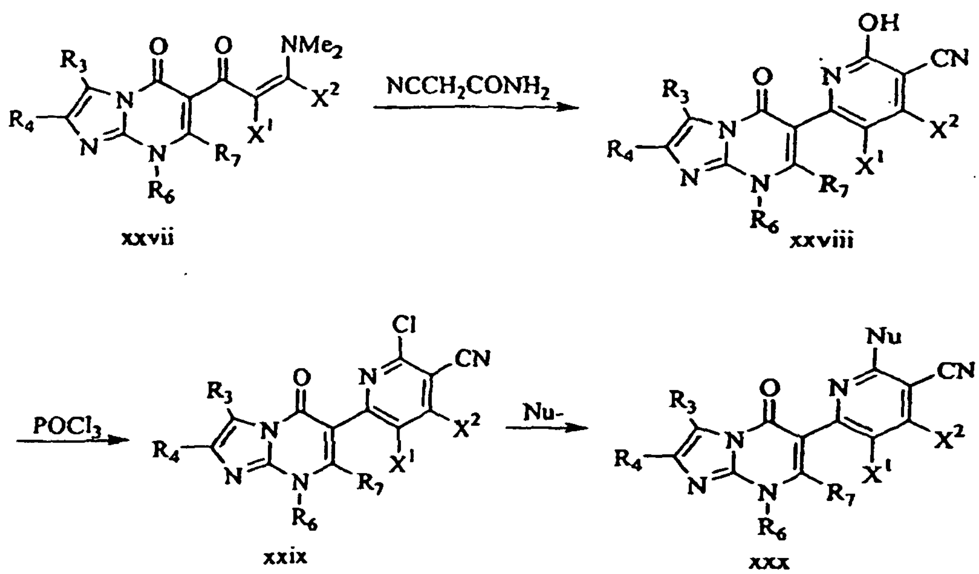


xxvii xxviii xxix xxx

Cyclization of compound (xxvii) (wherein $X^1$ and $X^2$ are substituents) with cyanoacetamide in the presence of a base such as potassium carbonate, potassium tert-butoxide, sodium methoxide in an appropriate solvent such as dioxane, ethanol, DMF at a temperature of 50-150°C for a period of 1-24 hours gives the pyridone (xxviii). Compound (xxviii) can be converted to the corresponding chloride (xxix) by reaction with $POCl_3$ with or without a solvent such as acetonitrile or toluene at a temperature of 25-120°C for a period of 1-24 hours. Compound (xxix) can be modified by reaction with a variety of nucleophiles ("$Nu^-$") such as mono or dialkylamine, alkoxide, thiol or carbon nucleophile to give compound (xxx).

## Reaction Scheme J

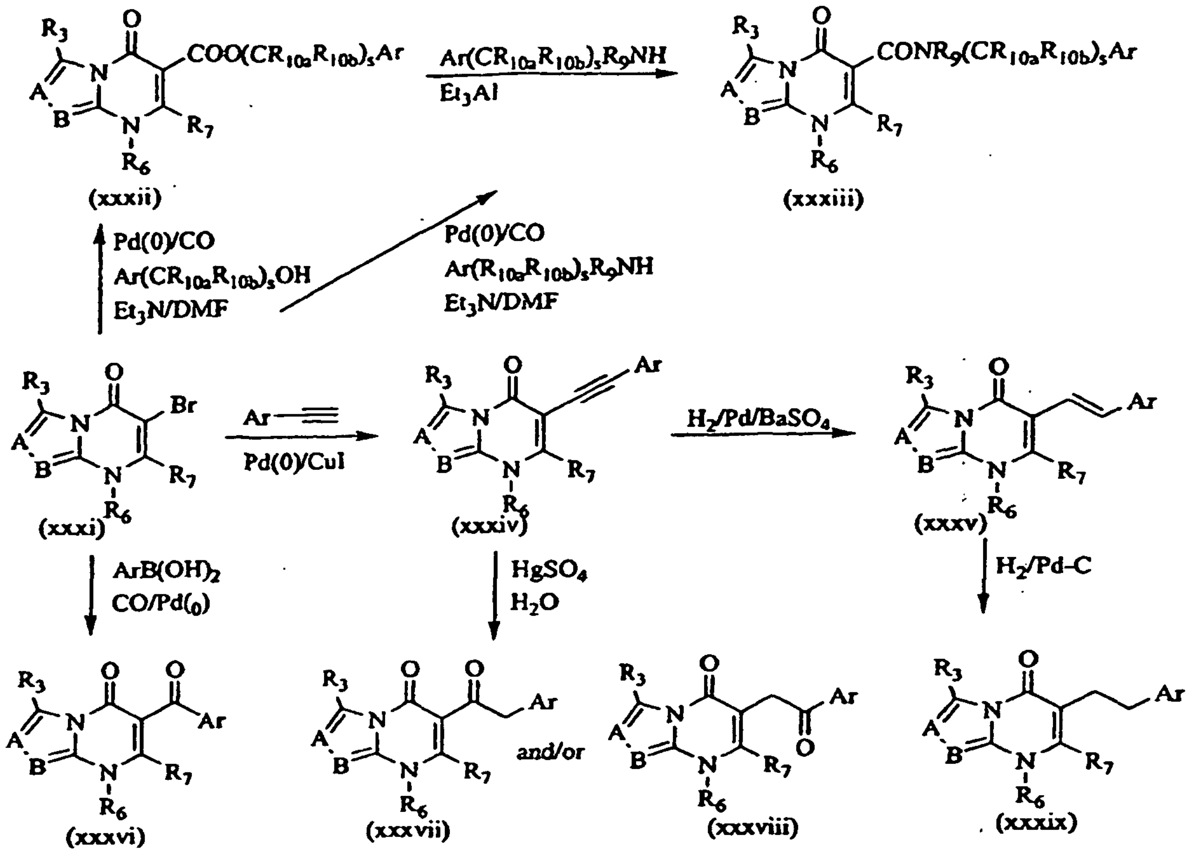


The bromo compound (xxxi) (see Reaction Scheme H) can be converted to the corresponding carbon analogs by various palladium catalyzed cross coupling reactions. Palladium catalyzed reaction of compound (xxxi) with carbon monoxide in the presence of an alcohol in a solvent such as alcohol or DMF at a temperature of 25-60°C for a period of 1-24 hours gives the ester (xxxii). Reaction of the ester (xxxii) with a primary or secondary amine and triethylaluminum in a solvent such as dichloromethane or toluene at a temperature of 0-100°C for a period of 1-16 hours gives the amide (xxxiii).

Palladium catalyzed coupling of compound (xxxi) with arylacetylene in the presence of CuI and a base such as triethylamine in a solvent such as triethylamine, dioxane or DMF at a temperature of 25-120°C for a period of 1-16 hours gives the alkyne (xxxiv). Selective hydrogenation of the alkyne (xxxiv) catalyzed by palladium/$BaSO_4$ under hydrogen atmosphere in a solvent such as methanol, ethyl acetate or DMF at room temperature for a period of 1-24 hours gives the alkene (xxxv). Further reduction with a catalyst such as palladium on carbon under hydrogen atmosphere in a solvent such as methanol, ethanol or ethyl acetate at room temperature for a period of 1-24 hours gives the corresponding alkane (xxxix).

Palladium catalyzed cross coupling of compound (xxxi) with an arylboronic acid in the presence of a base such as sodium carbonate, cesium carbonate or cesium fluoride in the carbon monoxide atmosphere in a solvent such as benzene, ethanol, water, DME or a mixture thereof at a temperature of 25-100°C for a period of 1-24 hours gives the ketone (xxxvi).

Reaction of the alkyne xxxiv with $HgSO_4$ in water at a temperature of 25-120°C for a period of 1-24 hours gives the corresponding ketone (xxxvii) and/or (xxxviii).

## Reaction Scheme K

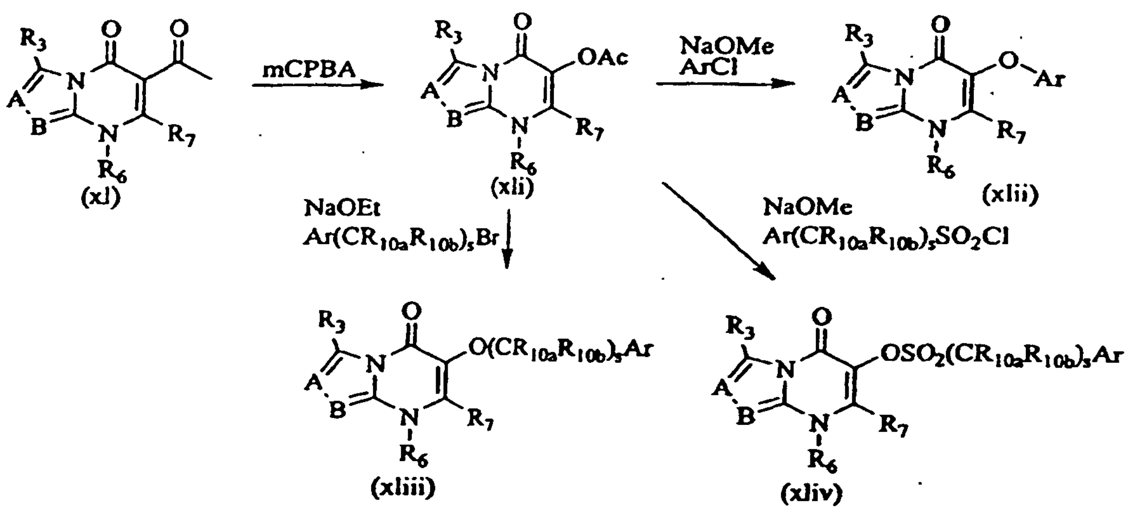


Oxidation of the ketone (xl) with m-chloroperoxybenzoic acid in a solvent such as dichloromethane or chloroform at a temperature of 0-60°C for a period of 1-72 hours gives the ester (xli). Reaction of compound (xli) with a reactive aryl halide, such as substituted or unsubstituted fluoronitrobenzene, 2- or 4-chloropyridine or 2- or 4-chloropyrimidine, in the presence of a base such as sodium methoxide or potassium t-butoxide in an inert solvent such as THF, DME or DMF at a temperature of 25-120°C for a period of 1-24 hours gives the aryloxy compound (xlii). Alkylation of compound (xli) with an aryl alkyl halide in the presence of a base such as sodium ethoxide or potassium hydroxide in as solvent such as THF, DMF or DMSO at a temperature for a period of 1-24 hours gives the corresponding alkoxy compound (xliii). Treatment of the ester (xli) with a base such as sodium methoxide in a solvent such as THF or DMF at room temperature for a period of 0.5 to 2 hours, followed by a sulfonyl chloride at a temperature of 0-60°C for a period of 1-16 hours gives the sulfonate (xliv).

## Reaction Scheme L

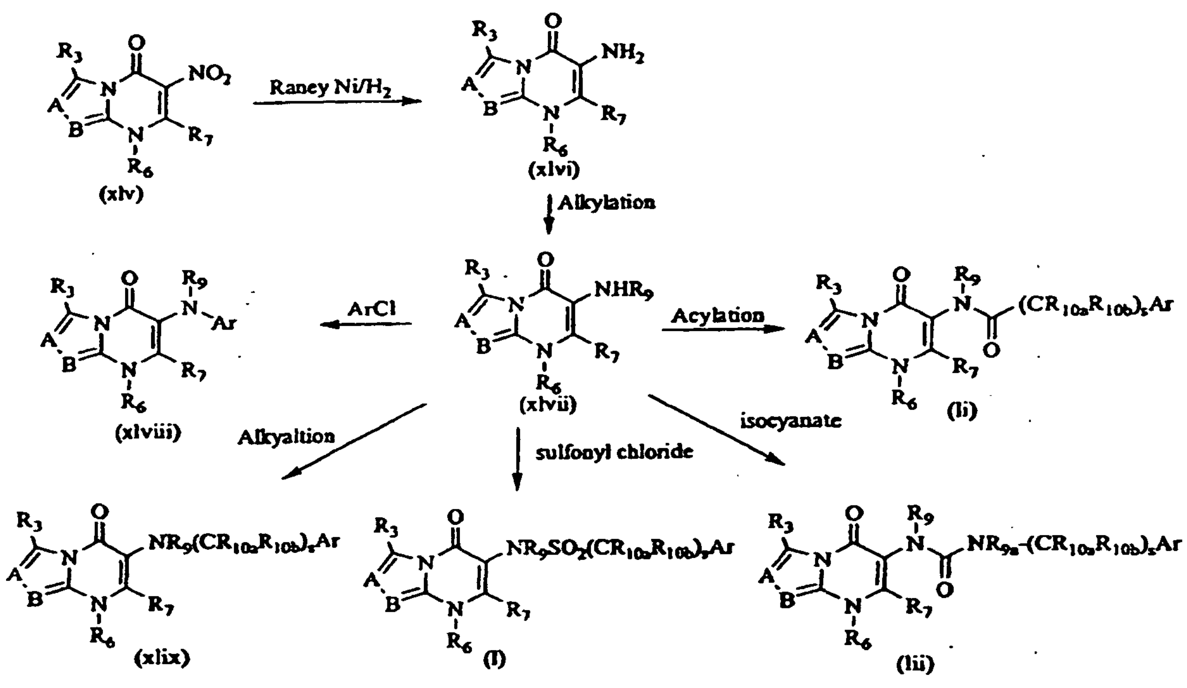

The nitro compound (xlv) can be reduced to the corresponding amine (xlvi) by 1) hydrogenation in the presence of a catalyst such as Raney nickel in a solvent such as methanol, ethanol or ethyl acetate at room temperature for a period of 1-24 hours; 2) chemical reduction such as $SnCl_2$ in a solvent such as water at a temperature of 25-100°C for a period of 1-24 hours. The amine (xlvi) can be alkylated with 1) an alkyl halide in the presence of a base such as potassium carbonate, triethylamine or sodium methoxide in an appropriate solvent such as acetonitrile, ethanol or chloroform at a temperature of 25-100°C for a period of 1-24 hours; 2) an aldehyde in the presence of a reducing agent such as sodium cyanoborohydride in a solvent such as methanol, dichloromethane or a mixture thereof at a temperature of 25-80°C for a period of 1-72 hours to give compound (xlvii). Reaction of the primary ($R^9$ = H) or secondary amine (xlvii) with a reactive aryl chloride such as chloronitrobenzene, chloropyridine or chloropyrimidine with or without a base such as potassium carbonate, triethylamine or sodium hydride in an appropriate solvent such as THF, DMF or dioxane at a temperature of 25-120°C for a period of 1-24 hours give the arylamino compound (xlviii). Further alkylation of (xlvii) with an arylalkyl halide with or without a base such as potassium carbonate, triethylamine or sodium hydride in an appropriate solvent such as acetonitrile, THF or DMF at a temperature of 25-100°C for a period of 1-24 hours give the amino compound (xlix). Reaction of amino compound (xlvii) with sulfonyl chloride in the presence of a base such as triethylamine, pyridine or potassium carbonate in a solvent such as dichloromethane, chloroform or benzene at a temperature of 25-100°C for a period of 1-24 hours gives the sulfonamide (1). Acylation of the amine (xlvii) with 1) an acid chloride in the presence of a base such as triethylamine, pyridine or potassium carbonate in an appropriate solvent such as dichloromethane, THF or DMF at a temperature of 25-100°Cfor a period of 1-16 hours; 2) an carboxylic acid with a coupling reagent such as DCC or EDC in an appropriate solvent such as chloroform or DMF for a period of 1-16 hours gives the corresponding amide (li). Reaction of the amine (xlvii) with an isocyanate in a solvent such as benzene, chloroform or dioxane at a temperature of 25-120°C for a period of 1-24 hours gives the urea compound (lii).

## Reaction Scheme M

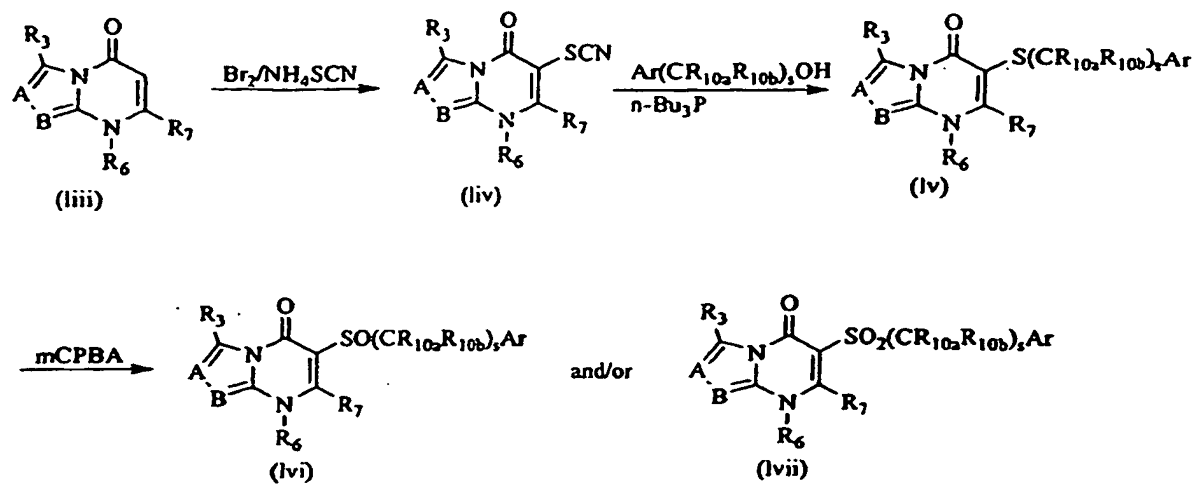


(liii) (liv) (lv)

(lvi) and/or (lvii)

Treatment of compound (liii) with bromine in the presence of ammonium thioisocyanate or potassium isothiocyanate in a solvent such as acetic acid, chloroform or ethanol at a temperature of 0-80°C for a period of 1-24 hours gives the thioisocyanate (liv). Alkylation of compound (liv) with an alcohol in the presence of tributylphosphine in a solvent such as benzene, chloroform or dioxane at a temperature of 25-120°C for a period of 1-24 hours gives the sulfide (lv). Oxidation of the sulfide (lv) with an oxidation reagent such as mCPBA or hydrogen peroxide in an appropriate solvent such as dichloromethane, ethanol, water or a mixture thereof at a temperature of 0-60°C for a period of 1-48 hours gives the corresponding sulfoxide (lvi) and sulfone (lvii).

Reaction Scheme N

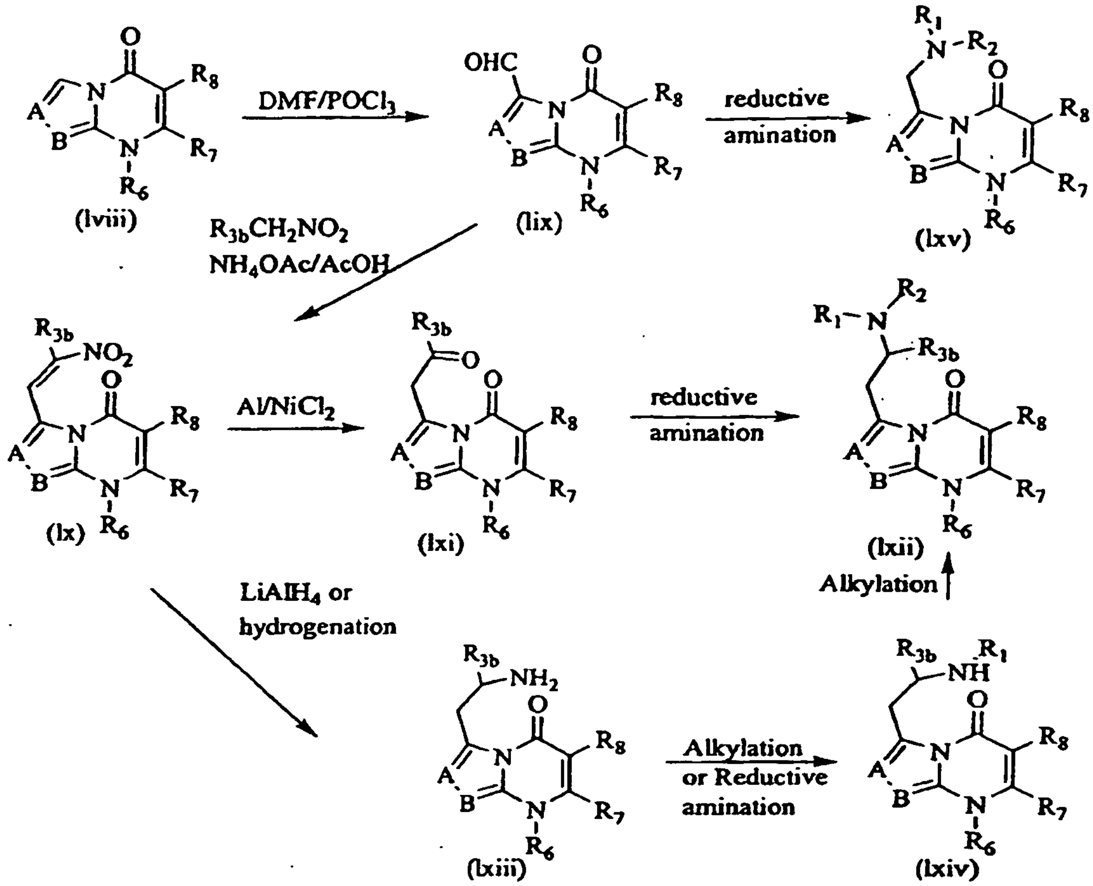


Compound (lviii) can be converted to the corresponding aldehyde (lix) by reaction with $POCl_3$ and DMF at a temperature of 0-120°C for a period of 1-24 hours. Reductive amination of the aldehyde (lix) with a primary or secondary amine in the presence of a reducing reagent such as sodium cyanoborohydride in an appropriate solvent such as methanol, dichloromethane, THF or a mixture thereof at a temperature of 0-80°C for a period of 1-24 hours gives the amine (lxv). Condensation of the aldehyde (lix) with nitroalkane in the presence of a base such as ammonium acetate in a solvent such as acetic acid at a temperature of 40-100°C for a period of 1-24 hours gives the nitroolefin (lx), which can be reduced to the corresponding carbonyl compound (lxi) with a reducing agent such as aluminum in the presence of a catalyst such as nickel chloride in a solvent such as DMF, THF or ethanol at a temperature of 25-100°C for a period of 1-24 hours. Reductive amination of carbonyl (lxi) with an amine and a reducing agent such as sodium cyanoborohydride in a solvent such as methanol, dichloromethane or mixture thereof at a temperature of 0-80°C for a period of 1-16 hours gives the amino compound (lxii). Alternatively, the nitroolefin (lx) can be reduced to the corresponding amine (lxiii) by using a reducing agent such as lithium aluminum hydride in an appropriate solvent such as ether or THF at a temperature of 0-80°C for a period of 1-16 hours, or by hydrogenation with a catalyst such as palladium on carbon under hydrogen atmosphere in a solvent such as methanol or ethyl acetate at room temperature for a period of 1-16 hours. The amine (lxiii) can be converted to the secondary amine (lxiv) or tertiary amine (lxii) by 1) alkylation with an alkyl halide in a solvent such as dichloromethane or ethyl acetate at a temperature of 0-80°C for a period of 1-16 hours; or 2) reductive amination with an aldehyde and a reducing agent such as sodium cyanoborohydride in a solvent such as methanol, dichloromethane or mixture thereof at a temperature of 0-80°C for a period of 1-16 hours.

Reaction Scheme O

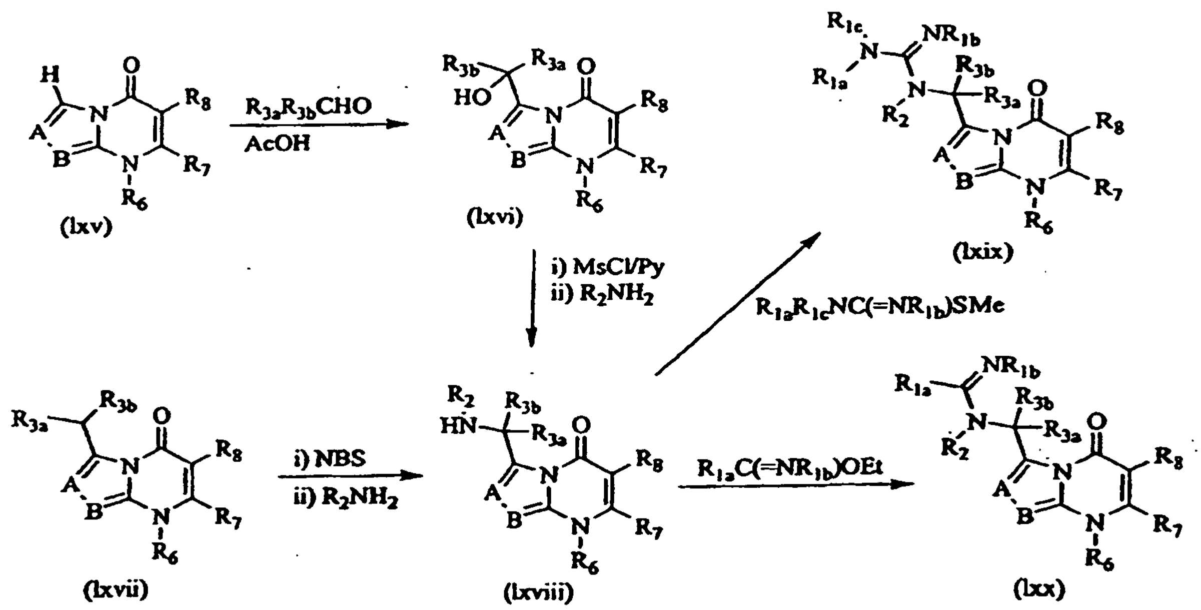


(lxv) (lxvi) (lxix) (lxvii) (lxviii) (lxx)

Compound (lxv) can be converted to the corresponding alcohol (lxvi) by reaction with an aldehyde with or without an acid catalyst such as hydrochloric acid, toluenesulfonic acid in an inert solvent such as ethanol, dioxane or acetic acid at a temperature of 25-120°C for a period of 1-24 hours. Alternatively, the alcohol (lxvi) may be formed first by condensation of an appropriate acid or acid chloride with starting (lxv) to give a ketone followed by reaction with a Grignard, or organolithium reagent. The alcohol (lxvi) can be further modified to the corresponding amine (lxviii) by reaction first with methanesulfonyl chloride in the presence of a base such as pyridine, triethylamine in an inert solvent such as dichloromethane, chloroform or pyridine at a temperature of 25-60°C for a period of 1-24 hours, followed by reaction with ammonium or a primary amine. Alternatively, bromination of compound (lxvii) with a brominating reagent such as N-succinamide in an inert solvent such as carbon tetrachloride or DMF at a temperature of 25-100°C for a period of 2-16 hours gives the corresponding bromide (lxviii) which reacts with an amine to give the amino compound (lxviii). The amino compound (lxviii) can be converted to guanidine derivative (lxix) by reaction with a S-methylthiourea in an appropriate solvent such as DMF, THF, ethanol or acetonitrile at a temperature of 25-120°C for a period of 1-24 hours. The amino compound (lxviii) can also be converted to the corresponding amidine derivative (lxx) by reaction with an imidate in an appropriate solvent such as ethanol, acetonitrile or DMF for a period of 2-24 hours.

## Reaction Scheme P

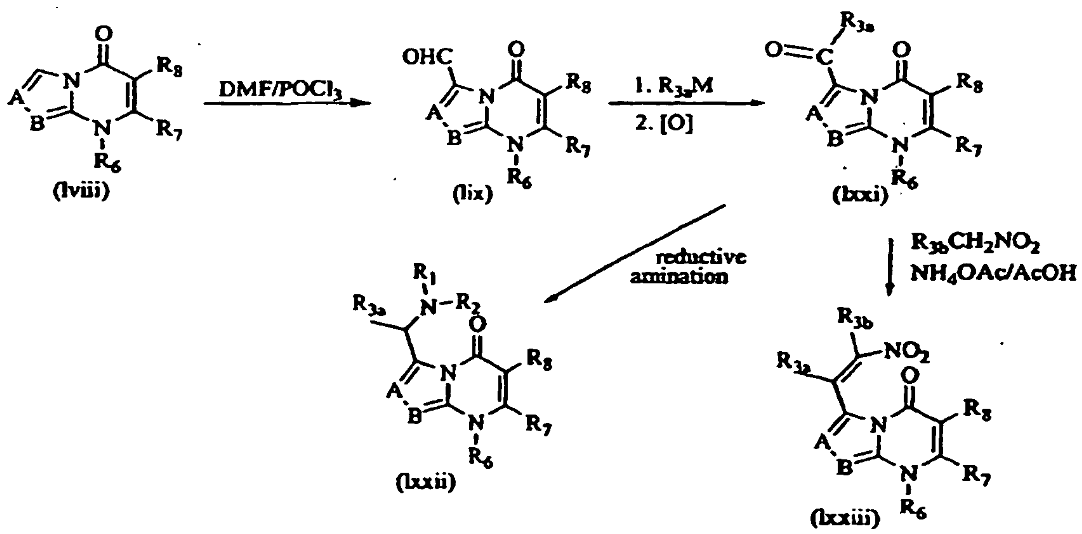


(lviii) (lix) (lxxi) (lxxii) (lxxiii)

Compound (lviii) can be converted to the corresponding aldehyde (lix) by reaction with $POCl_3$ and DMF at a temperature of 0-120°C for a period of 1-24 hours. Aldehyde (lix) may then form ketone (lxxi) first through reaction with an appropriate Grignard or organolithium reagent in a solvent such as THF or ethyl ether at a temperature of 78-60°C followed by an oxidation using Swern conditions or $MnO_2$ or PCC in a solvent such as methylene chloride at a temperature from 0-75°C.. Reductive amination of the ketone (lxxi) with a primary or secondary amine in the presence of a reducing reagent such as sodium cyanoborohydride in an appropriate solvent such as methanol, dichloromethane, THF or a mixture thereof at a temperature of 0-80°C for a period of 1-24 hours gives the amine (lxxii). Condensation of the aldehyde (lxxi) with nitroalkane in the presence of a base such as ammonium acetate in a solvent such as acetic acid at a temperature of 40-100°C for a period of 1-24 hours gives the nitroolefin (lxxiii).

The compounds of the present invention may generally be utilized as the free base. Alternatively, the compounds of this invention may be used in the form of acid addition salts. Acid addition salts of the free amino compounds of the present invention may be prepared by methods well known in the art, and may be formed from organic and inorganic acids. Suitable organic acids include maleic, fumaric, benzoic, ascorbic, succinic, methanesulfonic, acetic, trifluoroacetic, oxalic, propionic, tartaric, salicylic, citric, gluconic, lactic, mandelic, cinnamic, aspartic, stearic, palmitic, glycolic, glutamic, and benzenesulfonic acids. Suitable inorganic acids include hydrochloric, hydrobromic, sulfuric, phosphoric, and nitric acids. Thus, the term "pharmaceutically acceptable salt" of structure (I) is intended to encompass any and all acceptable salt forms.

With regard to stereoisomers, the compounds of structure (I) may have chiral centers and may occur as racemates, racemic mixtures and as individual enantiomers or diastereomers. All such isomeric forms are included within the present invention, including mixtures thereof. Furthermore, some of the crystalline forms of the compounds of structure (I) may exist as polymorphs, which are included in the present invention. In addition, some of the compounds of structure (I) may also form solvates with water or other organic solvents. Such solvates are similarly included within the scope of this invention.

The effectiveness of a compound as a GnRH receptor antagonist may be determined by various assay methods. Suitable GnRH antagonists of this invention are capable of inhibiting the specific binding of GnRH to its receptor and antagonizing activities associated with GnRH. For example, inhibition of GnRH stimulated LH release in immature rats may be measured according to the method of Vilchez-Martinez *(Endocrinology 96*:1130-1134, 1975). Briefly, twenty-five day old male Spraque-Dawley rats are administered an GnRH antagonist in saline or other suitable formulation by oral gavage, subcutaneous injection, or intravenous injection. This is followed by subcutaneous injection of 200 ng GnRH in 0.2 ml saline. Thirty minutes after the last injection, the animals are decapitated and trunk blood collected. After centrifugation, the separated plasma is stored at -200°C until determination of the LH and FSH by radioimmunoassay. Other techniques for determining the activity of GnRH receptor antagonists are well known in the field, such as the use of cultured pituitary cells for measuring GnRH activity (Vale et al., *Endocrinology 91*:562-572, 1972), and a technique for measuring radioligand binding to rat pituitary membranes (Perrin et al., *Mol. Pharmacol. 23*:44-51, 1983).

Activity of GnRH receptor antagonists are typically calculated from the $IC_{50}$ as the concentration of a compound necessary to displace 50% of the radiolabeled ligand from the GnRH receptor, and is reported as a "$K_i$" value calculated by the following equation:

$$K_i = \frac{IC_{50}}{1 + L / K_D}$$

where L = radioligand and $K_D$ = affinity of radioligand for receptor (Cheng and Prusoff, *Biochem. Pharmacol*. *22*:3099, 1973). GnRH receptor antagonists of this invention have a $K_i$ of 100 μM or less. In a preferred embodiment of this invention, the GnRH receptor antagonists have a $K_i$ of less than 10 μM, and more preferably less than 1μM. More preferred compounds include: 1-1, 1-2-5, 12, 13-15, 19, 21, 27, 31, 32, 34, 37, 55, 58, 64, 73, 2B, 2D, 3A, 3B, 3D, 4A, 4B, 4C, 4D, 4G, 4H, 5A and 5B *(see* Examples below).

Representative GnRH receptor antagonists of this invention include the following compounds:

(a) 3-(N-Benzyl-N-methyl)aminomethyl-2-(tert-butyl)-6-methyl-7-(2-fluorobenzyl)-5-(3-methoxyphenyl)imidazolo[1,2-a]pyrimid-4-one
(b) 3-(N-(2-Pyridylmethyl))aminomethyl-2-(tert-butyl)-6-methyl-7-(2-fluorobenzyl)-5-(3-methoxyphenyl)imidazolo[1,2-a]pyrimid-4-one
(c) 3-(N-(2-Pyridylmethyl)-N-methyl)aminomethyl-2-(tert-butyl)-6-methyl-7-(2-fluorobenzyl)-5-(3-methoxyphenyl)imidazolo[1,2-a]pyrimid-4-one
(d) 3-[N-Methyl-N-(2-pyridylethyl)]aminomethyl-2-(tert-butyl)-6-methyl-7-(2-fluorobenzyl)-5-(3-methoxyphenyl)imidazolo[1,2-a]pyrimid-4-one
(e) 3-[N-(2-Furanmethyl)-N-methyl]aminomethyl-2-(tert-butyl)-6-methyl-7-(2-fluorobenzyl)-5-(3-methoxyphenyl)imidazolo[1,2-a]pyrimid-4-one
(f) 3-[N-Methyl-N-(2-pyridylethyl)]aminomethyl-2-(ethoxycarbonylmethyl)-6-methyl-7-(2-fluorobenzyl)-5-(3-methoxyphenyl)imidazolo[1,2-a]pyrimid-4-one
(g) 1-(N-Benzyl-N-methyl)aminomethyl-2-(tert-butyl)-4-(2-fluorobenzyl)-6-(3-phenylpropylaminocarbonyl)pyrrolo[1,2-a]pyrimid-7-one
(h) 1-[(N-Methyl-N-(2-pyridylethyl)]aminomethyl-2-(tert-butyl)-4-(2-fluorobenzyl)-6-(3-phenylpropylaminocarbonyl)pyrrolo[1,2-a]pyrimid-7-one
(i) 1-[(N-Methyl-N-(2-pyridylethyl)]aminomethyl-2-(tert-butyl)-4-(2-fluorobenzyl)-5-methyl-6-(3-methoxyphenyl)pyrrolo[1,2-a]pyrimid-7-one
(j) 1-(N-Benzyl-N-methyl)aminomethyl-2-(t-butyl)-4-(2-fluorobenzyl)-5-methyl-6-(3-methoxyphenyl)imidazolo[3,4-a]pyrimid-7-one
(k) 1-[N-Methyl-N-(2-pyridylethyl)]aminomethyl-4-(2-fluorobenzyl)-5-methyl-6-(3-methoxyphenyl)imidazolo[3,4-a]pyrimid-7-one
(l) 1-[N-(2-Furanmethyl)-N-methyl]aminomethyl-4-(2-fluorobenzyl)-5-methyl-6-(3-methoxyphenyl)imidazolo[3,4-a]pyrimid-7-one
(m) 3-(N-Benzyl-N-methyl)aminomethyl-6-methyl-7-(2-fluorobenzyl)-5-(3-methoxyphenyl)-1,2,4-triazolo[4,3-a]pyrimid-4-one
(n) 3-[N-Methyl-N-(2-pyridylethyl)]aminomethyl-6-methyl-7-(2-fluorobenzyl)-5-(3-methoxyphenyl)-1,2,4-triazolo[4,3-a]pyrimid-4-one
(o) 3-[N-(2-Furanmethyl)-N-methyl]aminomethyl-6-methyl-7-(2-fluorobenzyl)-5-(3-methoxyphenyl)-1,2,4-triazolo[4,3-a]pyrimid-4-one
(p) 1-(N-Benzyl-N-methyl)aminomethyl-2-(1-methoxycarbonyl-1-methylethyl)-7-(2-fluorobenzyl)-6-methyl-5-(3-methoxyphenyl)imidazolo[1,2-a]pyrimid-4-one
(q) 1-[N-(2-Pyridylethyl)-N-methyl]aminomethyl-2-(1-methoxycarbonyl-1-methylethyl)-7-(2-fluorobenzyl)-6-methyl-5-(3-methoxyphenyl)imidazolo[1,2-a]pyrimid-4-one

As mentioned above, the GnRH receptor antagonists of this invention have utility over a wide range of therapeutic applications, and may be used to treat a variety of sex-hormone related conditions in both men and women, as well as mammals in general. For example, such conditions include endometriosis, uterine fibroids, polycystic ovarian disease, hirsutism, precocious puberty, gonadal steroid-dependent neoplasia such as cancers of the prostate, breast and ovary, gonadotrophe pituitary adenomas, sleep apnea, irritable bowel syndrome, premenstrual syndrome, benign prostatic hypertrophy, contraception and infertility (e.g., assisted reproductive therapy such as *in vitro* fertilization).

The compounds of this invention are also useful as an adjunct to treatment of growth hormone deficiency and short stature, and for the treatment of systemic lupus erythematosis.

In addition, the compounds are useful in combination with androgens, estrogens, progesterones, and antiestrogens and antiprogestogens for the treatment of endometriosis, fibroids, and in contraception, as well as in combination with an angiotensin-converting enzyme inhibitor, an angiotensin II-receptor antagonist, or a renin inhibitor for the treatment of uterine fibroids. The compounds may also be used in combination with bisphosphonates and other agents for the treatment and/or prevention of disturbances of calcium, phosphate and bone metabolism, and in combination with estrogens, progesterones and/or androgens for the prevention or treatment of bone loss or hypogonadal symptoms such as hot flashes during therapy with a GnRH antagonist.

In another embodiment of the invention, pharmaceutical compositions containing one or more GnRH receptor antagonists are disclosed. For the purposes of administration, the compounds of the present invention may be formulated as pharmaceutical compositions. Pharmaceutical compositions of the present invention comprise a GnRH receptor antagonist of the present invention and a pharmaceutically acceptable carrier and/or diluent. The GnRH receptor antagonist is present in the composition in an amount which is effective to treat a particular disorder—that is, in an amount sufficient to achieve GnRH receptor antagonist activity, and preferably with acceptable toxicity to the patient. Typically, the pharmaceutical compositions of the present invention may include a GnRH receptor antagonist in an amount from 0.1 mg to 250 mg per dosage depending upon the route of administration, and more typically from I mg to 60 mg. Appropriate concentrations and dosages can be readily determined by one skilled in the art.

Pharmaceutically acceptable carrier and/or diluents are familiar to those skilled in the art. For compositions formulated as liquid solutions, acceptable carriers and/or diluents include saline and sterile water, and may optionally include antioxidants, buffers, bacteriostats and other common additives. The compositions can also be formulated as pills, capsules, granules, or tablets which contain, in addition to a GnRH receptor antagonist, diluents, dispersing and surface active agents, binders, and lubricants. One skilled in this art may further formulate the GnRH receptor antagonist in an appropriate manner, and in accordance with accepted practices, such as those disclosed in *Remington's Pharmaceutical Sciences,* Gennaro, Ed., Mack Publishing Co., Easton, PA 1990.

In another embodiment, the present invention provides a method for treating sex-hormone related conditions as discussed above. Such methods include administering of a compound of the present invention to a warm-blooded animal in an amount sufficient to treat the condition. In this context, "treat" includes prophylactic administration. Such methods include systemic administration of a GnRH receptor antagonist of this invention, preferably in the form of a pharmaceutical composition as discussed above. As used herein, systemic administration includes oral and parenteral methods of administration. For oral administration, suitable pharmaceutical compositions of GnRH receptor antagonists include powders, granules, pills, tablets, and capsules as well as liquids, syrups, suspensions, and emulsions. These compositions may also include flavorants, preservatives, suspending, thickening and emulsifying agents, and other pharmaceutically acceptable additives. For parental administration, the compounds of the present invention can be prepared in aqueous injection solutions which may contain, in addition to the GnRH receptor antagonist, buffers, antioxidants, bacteriostats, and other additives commonly employed in such solutions.

Rat Anterior Pituitary Cell Culture Assay of GnRH Antagonists

Anterior pituitary glands are collected from 7-week-old female Sprague-Dawley rats and the harvested glands digested with collagenase in a dispersion flask for 1.5 hr at 37°C. After collagenase digestion, the glands are further digested with neuraminidase for 9 min at 37°C. The digested tissue is then washed with 0.1% BSA/McCoy's 5A medium, and the washed cells suspended in 3% FBS/0.1 BSA/McCoy's 5A medium and plated into 96-well tissue culture plates at a cell density of 40,000 cells per well in 200 µl medium. The cells are then incubated at 37°C for 3 days. One pituitary gland normally yields one 96-well plate of cells, which can be used for assaying three compounds. For assay of an GnRH antagonist, the incubated cells are first washed with 0.1% BSA/McCoy's 5A medium once, followed by addition of the test sample plus 1nM GnRH in 200 µl 0.1% BSA/McCoy's 5A medium in triplicate wells. Each sample is assayed at 5-dose levels to generate a dose-response curve for determination of its potency on the inhibition of GnRH stimulated LH and/or FSH release. After 4-hr incubation at 37°C, the medium is harvested and the level of LH and/or FSH secreted into the medium determined by RIA.

RIA of LH and FSH

For determination of the LH levels, each sample medium is assayed in duplicates and all dilutions are done with RIA buffer (0.01M sodium phosphate buffer/0.15M NaCl/1% BSA/0.01% NaN3, pH 7.5) and the assay kit is obtained from the Nation Hormone and Pituitary Program supported by NIDDK. To a 12x75 mm polyethylene test tube is added 100 µl of sample medium diluted 1:5 or rLH standard in RIA buffer and 100 µl of [125I]-labeled rLH (~30,000 cpm) plus 100 µl of rabbit anti-rLH antibody diluted 1:187,500 and 100 µl RIA buffer. The mixture is incubated at room temperature over-night. In the next day, 100 µl of goat anti-rabbit IgG diluted 1:20 and 100 µl of normal rabbit serum diluted 1:1000 are added and the mixture incubated for another 3 hr at room temperature. The incubated tubes are

then centrifuged at 3,000 rpm for 30 min and the supernatant removed by suction. The remaining pellet in the tubes is counted in a gamma-counter. RIA of FSH is done in a similar fashion as the assay for LH with substitution of the LH antibody by the FSH antibody diluted 1:30,000 and the labeled rLH by the labeled rFSH.

Radio-iodination of GnRH peptide

The GnRH analog is labeled by the chloramine-T method. To 10 μg of peptide in 20 μl of 0.5M sodium phosphate buffer, pH 7.6, is added 1 mCi of Na125I, followed by 22.5 μg chloramine-T and the mixture vortexed for 20 sec. The reaction is stopped by the addition of 60 μg sodium metabisulfite and the free iodine is removed by passing the iodinated mixture through a C-8 Sep-Pak cartridge (Millipore Corp., Milford, MA). The peptide is eluted with a small volume of 80% acetonitrile/water. The recovered labeled peptide is further purified by reverse phase HPLC on a Vydac C-18 analytical column (The Separations Group, Hesperia, CA) on a Beckman 334 gradient HPLC system using a gradient of acetonitrile in 0.1% TFA. The purified radioactive peptide is stored in 0.1 % BSA/20% acetonitrile/0.1% TFA at -800C and can be used for up to 4 weeks.

GnRH receptor membrane binding assay

Cells stably, or transiently, transfected with GnRH receptor expression vectors are harvested, resuspended in 5% sucrose and homogenized using a polytron homogenizer (2x 15 sec). Nuclei are removed by centrifugation (3000 x g for 5 min.), and the supernatant centrifuged (20,000 x g for 30 min, 4° C) to collect the membrane fraction. The final membrane preparation is resuspended in binding buffer (10mM Hepes (pH 7.5), 150 mM NaCl, and 0.1% BSA) and stored at -70°C. Binding reactions are performed in a Millipore MultiScreen 96-well filtration plate assembly with polyethylenimine coated GF/C membranes. The reaction is initiated by adding membranes (40 ug protein in 130 ul binding buffer) to 50ul of [$^{125}$I]-labeled GnRH peptide (~100,000 cpm), and 20ul of competitor at varying concentrations. The reaction is terminated after 90 minutes by application of vacuum and washing (2X) with phosphate buffered saline. Bound radioactivity is measured using 96-well scintillation counting (Packard Topcount) or by removing the filters from the plate and direct gamma counting. $K_i$ values are calculated from competition binding data using non-linear least squares regression using the Prism software package (GraphPad Software).

The following examples are provided for purposes of illustration. In summary, the GnRH receptor antagonists of this invention may be assayed by the methods disclosed above, while Examples 1-4 disclose the synthesis of representative compounds of this invention.

EXAMPLE 1

SYNTHESIS OF 2-(TERT-BUTYL)-3-[N-METHYL-N-(2-PYRIDYLETHYL)AMINOMETHYL]-5-(3-METHOXYPHENYL)-6-METHYL-7-(2-FLUOROBENZYL)IMMIDAZOLO[1,2-A]PYRIMID-4-ONE

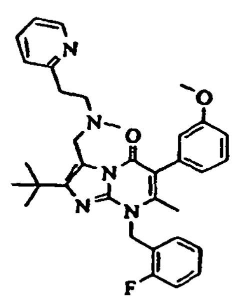

Step 1A 5-Bromo-6-methyl-2-(tert-butyl)-7*H*-imidazolo[1,2-a]pyrimid-4-one

Under nitrogen atmosphere, to a solution of 2-amino-5-bromo-4-hydroxy-6-methylpyrimidine (4.08 g, 20 mmol.) in dry DMF (80 ml), sodium hydride (800 mg, 20 mmol., 60% in mineral oil) was carefully added. The mixture was stirred at room temperature for 0.5 hour, followed by addition of bromomethyl tert-butyl ketone (20 mmol.). The mixture was then stirred at room temperature overnight. The resultant mixture was concentrated in vacuo, and the residue was dissolved in acetone (50 ml) and diluted with water (100 ml). Slowly concentration under reduced pressure resulted in a precipitation. The solid was collected by filtration, washing with water, ether, and dried to yield the desired

product as a white powder (4.16 g, 80% yield); MS :284/286 $(M+H)^+$.

Step 1B 5-(3-methoxyphenyl)-6-methyl-2-(tert-butyl)-7*H*-imidazolo[1,2-a]pyrimid-4-one

To a pressure vessel , potassium carbonate (1.38 g, 10 mmol.) , 3-methoxyphenylboronic acid (1.06 g, 7.0 mmol.), 5-bromo-6-methyl-2-(tert-butyl)-7*H*-imidazolo[1,2-a]pyrimid-4-one (1.42 g, 5 mmol), toluene (20 ml), water (5 ml) were added. The mixture was bubbled by nitrogen gas for 10 minutes to remove the air. Then $Pd(PPh_3)_4$ (500 mg) was added and the vessel was sealed immediately and heated at 110°C for 6 hours. After cooled to room temperature, the mixture was extracted with ethyl acetate (100 ml). The ethyl acetate layer was washed with water, dried and concentrated to give a yellow solid. It was stirred with a mixture of ether /ethyl acetate (50ml/5ml) and solid was collected by filtration to give the crude product (0.74 g); MS : 312 $(M+H)^+$.

Step 1C 5-(3-methoxyphenyl)-6-methyl-2-(tert-butyl)-7-(2-fluorophenylmethyl)-imidazolo[1,2-a]pyrimid-4-one

To a solution of 5-(3-methoxyphenyl)-6-methyl-2-(tert-butyl)-7*H*-imidazolo[1,2-a]pyrimid-4-one (0.74 g, 2.4 mmol.) in DME (5 ml), tetrabutylammonium fluoride (4 ml, 1.0 M in THF) was added and followed by addition of 2-fluorobenzyl bromide (0.45 ml, 1.5 eq.). The mixture was stirred at room temperature overnight and then concentrated and purified by silica gel chromatography (hexane/ethyl acetate) to give the desired product (0.55 g) as a white powder; MS: 420$(M+H)^+$; NMR ($CDCl_3$, δ): 7.38-6.77 (9H, 4m), 5.69 (2H, s), 3.80(3H, s), 2.20 (3H, s), 1.32 (9H, s).

Step 1D 3-{N-methyl-N-[2-(2-pyridyl)ethyl)aminomethyl}-5-(3-methoxyphenyl)-6-methyl-2-(tert-butyl)-7-(2-fluorophenylmethyl)-imidazolo[1,2-a]pyrimid-4-one

To a solution of N-[2-(2-pyridylethyl)]-N-methylamine (136 mg, 1.0 mmol.) and aqueous formaldehyde (0.1 ml) in acetic acid (2 ml), 5-(3-methoxyphenyl)-6-methyl-2-(tert-butyl)-7-(2-fluorophenylmethyl)-imidazolo[1,2-a]pyrimid-4-one (210mg, 0.5mmol) was added. The solution was stirred at room temperature for 1 hours and directly purified by prep HPLC to give the pure product (240 mg) as a trifluoroacetic acid salt; MS :568$(M+H)^+$, 432. NMR (DMSO-$d_6$, δ): 9.16 (1H, brs), 8.21 (1H, d), 7.80 (1H, t), 7.39-6.86 (10H, m), 5.65 (2H, s), 4.85 (2H, s), 3.78 (3H, s), 3.66 (2H, brs), 3.28 (2H, t), 2.97 (3H, s), 2.27 (3H, s), 1.35 (9H; s).

Following the procedure similar to that described above, the following compounds were prepared:

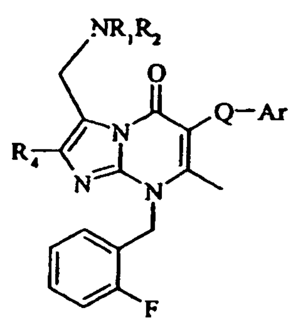

| Example | $R_1NR_2$ | $R_4$ | -Q-Ar | $MS(M+H)^+$ |
|---|---|---|---|---|
| 1-1 | 2-$PyCH_2CH_2NMe$ | t-Bu | 3-OMe-Ph | 568 |
| 1-2 | BnNMe | t-Bu | 3-OMe-Ph | 553 |
| 1-3 | Furyl$CH_2$Nme | t-Bu | 3-OMe-Ph | 543 |
| 1-4 | $Me_2NCH_2CH_2NMe$ | t-Bu | 3-OMe-Ph | 534 |
| 1-5 | $MeOCH_2CH_2NMe$ | t-Bu | 3-OMe-Ph | 521 |
| 1-6 | 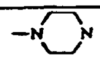 | t-Bu | 3-OMe-Ph | 532 |
| 1-7 | 2-$PyCH_2CH_2NMe$ | i-Bu | 3-OMe-Ph | 568 |
| 1-8 | BnNMe | i-Bu | 3-OMe-Ph | 553 |
| 1-9 | 2-$PyCH_2CH_2NMe$ | Me | 3-OMe-Ph | 526 |
| 1-10 | BnNMe | Me | 3-OMe-Ph | 511 |
| 1-11 | 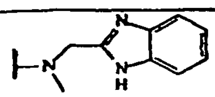 | t-Bu | 3-OMe-Ph | 593 |

| | | | | |
|---|---|---|---|---|
| 1-12 | 2-$PyCH_2Nme$ | t-Bu | 3-OMe-Ph | 554 |
| 1-13 | 2-$PyCH_2CH_2NMe$ | 2-OH-t-Bu | 3-OMe-Ph | |
| 1-14 | $PhCH_2Nme$ | t-Bu | Ph | 523 |
| 1-15 | 2-$PyCH_2CH_2NMe$ | t-Bu | 2-Py | 538 |
| 1-16 | $PhCH_2Nme$ | t-Bu | Ph | 524 |
| 1-17 | BuNMe | t-Bu | Ph | |
| 1-18 | 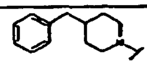 | t-Bu | Ph | |
| 1-19 | 2-$FurylCH_2NMe$ | t-Bu | Ph | 513 |
| 1-20 | 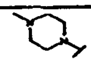 | t-Bu | Ph | 502 |
| 1-21 | $PhCH_2Nme$ | 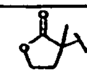 | 3-OMe-Ph | 595 |
| 1-22 | $PhCH_2NCH_2CH_2CN$ | 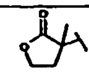 | 3-OMe-Ph | 634 |
| 1-23 | 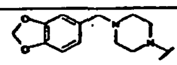 | 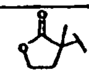 | 3-OMe-Ph | 694 |
| 1-24 | 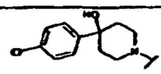 | 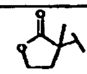 | 3-OMe-Ph | 685 |
| 1-25 | 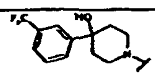 | 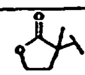 | 3-OMe-Ph | 719 |
| 1-26 | 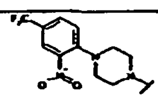 | 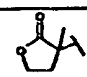 | 3-OMe-Ph | 749 |
| 1-27 | 2-$FurylCH_2NMe$ | 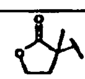 | 3-OMe-Ph | 585 |
| 1-28 | $Me_2NCH_2CH_2NMe$ | 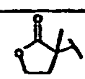 | 3-OMe-Ph | 576 |
| 1-29 | 2-$PyCH_2NH$ | 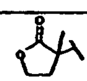 | 3-OMe-Ph | 582 |

| | | | | |
|---|---|---|---|---|
| 1-30 | $PhCH_2Nme$ | t-Bu | 3-Py | 524 |
| 1-31 | $PhCH_2Nme$ | t-Bu | 3-AcPh | 565 |
| 1-32 | $2\text{-}PyCH_2CH_2NMe$ | t-Bu | 3-AcPh | 580 |
| 1-33 | $2\text{-}FurylCH_2NMe$ | t-Bu | 3-AcPh | 555 |
| 1-34 | $2\text{-}PyCH_2NH$ | t-Bu | 3-OMe-Ph | 552 |
| 1-35 | $Me_2NCH_2CH_2NMe$ | t-Bu | 3-OMe-Ph | 546 |
| 1-36 | 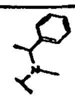 | t-Bu | 3-OMe-Ph | 567 |
| 1-37 | 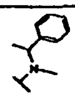 | t-Bu | 3-OMe-Ph | 567 |
| 1-38 | 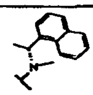 | t-Bu | 3-OMe-Ph | 617 |
| 1-39 | 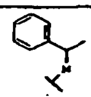 | t-Bu | 3-OMe-Ph | 553 |
| 1-40 | 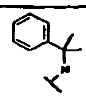 | t-Bu | 3-OMe-Ph | 567 |
| 1-41 | 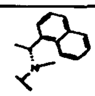 | t-Bu | 3-OMe-Ph | 432, 603 |
| 1-42 | 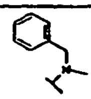 | t-Bu | 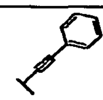 | 456, 577 |
| 1-43 | 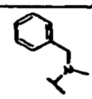 | t-Bu | 2-F-Bn | 434, 555 |
| 1-44 | 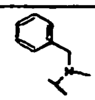 | t-Bu | 2-F-Ph | 541 |
| 1-45 | 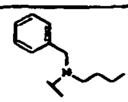 | t-Bu | 2-F-Ph | 583 |

| | | | | |
|---|---|---|---|---|
| 1-46 | 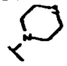 | t-Bu | 2-F-Ph | 523 |
| 1-47 | 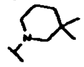 | t-Bu | 2-F-Ph | 533 |
| 1-48 | 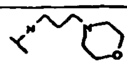 | t-Bu | 2-F-Ph | 564 |
| 1-49 | 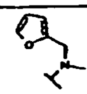 | t-Bu | 2-F-Ph | 531 |
| 1-50 | 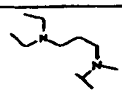 | t-Bu | 2-F-Ph | 564 |
| 1-51 | 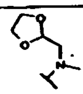 | t-Bu | 2-F-Ph | 537 |
| 1-52 | 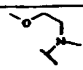 | t-Bu | 2-F-Ph | 509 |
| 1-53 | 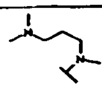 | t-Bu | 2-F-Ph | 536 |
| 1-54 | $2\text{-PyCH}_2\text{CH}_2\text{NMe}$ | t-Bu | 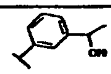 | 582 |
| 1-55 | $2\text{-PyCH}_2\text{CH}_2\text{NMe}$ | t-Bu | 2-F-Ph | 556 |
| 1-56 | 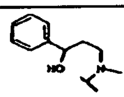 | t-Bu | 2-F-Ph | 420 |
| 1-57 | $2\text{-PyCH}_2\text{CH}_2\text{NMe}$ | t-Bu | 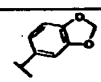 | 446 |
| 1-58 | $2\text{-PyCH}_2\text{Nme}$ | t-Bu | 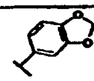 | 446 |
| 1-59 | 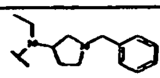 | t-Bu | 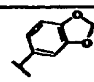 | 446 |
| 1-60 | 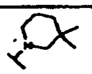 | t-Bu | 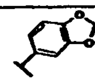 | 559 |
| 1-61 | 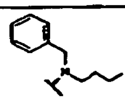 | t-Bu | 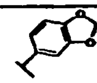 | 446 |
| 1-62 | 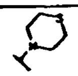 | t-Bu | 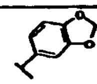 | 446 |

| | | | | |
|---|---|---|---|---|
| 1-63 | 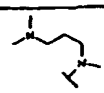 | t-Bu | 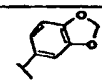 | 562 |
| 1-64 | 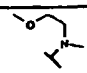 | t-Bu | 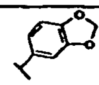 | 446 |
| 1-65 | 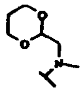 | t-Bu | 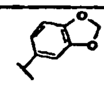 | 446 |
| 1-66 | 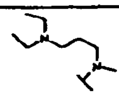 | t-Bu | 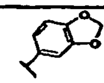 | 590 |
| 1-67 | 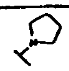 | t-Bu | 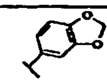 | 446 |
| 1-68 | 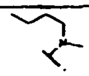 | t-Bu | 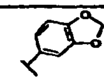 | 446 |
| 1-69 | 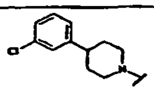 | t-Bu | 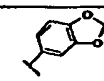 | 446 |
| 1-70 | 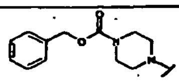 | t-Bu | 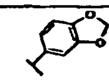 | 446 |
| 1-71 | 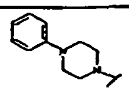 | t-Bu | 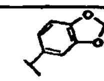 | 446 |
| 1-72 | 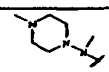 | t-Bu | 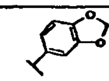 | 446 |
| 1-73 | $Et_2NCH_2CH_2NMe$ | t-Bu | 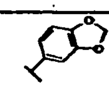 | 576 |

EXAMPLE 2.1

SYNTHESIS OF 1-(N-BENZYL-N-METHYLAMINOMETHYL)-2-(TERT-BUTYL)-4-(2-FLUOROBENZYL)-6-ETHOXYCARBONYLPYRROLO[1,2-A]PYRIMID-7-ONE (INTERMEDIATE)

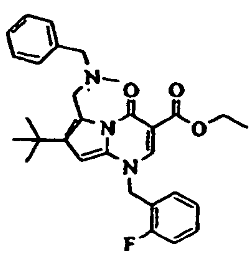

Step 2A 5-Ethoxycarbonyl-2-methyl-3-(2-oxo-3,3-dimethyl-butyl)pyrimid-4-one

To a suspension of 5-ethoxycarbonyl-2-methylpyrimid-4-one (2.7g, 14.75 mmol.) in DME (20 ml), tetrabutylammonium fluoride (22 ml, 22.0 mmol.) was added . The solution was stirred at room temperature until solids dissolved , then 1-bromopinacolone (2.2 ml, 1.1 eq., 16.22 mmol) was added. The solution was stirred overnight and concentrated to a brown oil. The crude mixture was purified by silica gel column chromatography (hexane /ethyl acetate, 100/0 to 0/100). A less polar O-alkylated by-product was eluted first (1.8 g) and then the desired N-alkylated product (1.1 g); MS (281, M+H)$^+$. NMR ($CDCl_3$ ,δ): 8.57 (1H, s) , 5.06( 1H, s), 4.35(2H, q), 2.42 (3H, s), 1.34 (3H, t), 1.30 (9H,s ).

Step 2B 6-Ethoxycarbonyl-2-(tert-butyl)-4*H*-pyrrolo[1,2-a]pyrimid-7-one

5-Ethoxycarbonyl-2-methyl-3-(2-oxo-3,3-dimethyl-butyl)pyrimid-4-one (1.1 g, 3.9 mmol.) was added into sodium ethoxide solution, made *in situ* from sodium (200 mg) and dry ethanol (50 ml). After stirred for 2 hours, the mixture was acidified slowly with 6N HCl resulting a precipitation. The precipitates were collected by filtration and washed with water (20 ml x2), ether (20 ml x3), dried to give the desired product (0.8 g); MS (263, M+H)$^+$.

Step 2C 6-Ethoxycarbonyl-2-(tert-butyl)-4-(2-fluorobenzyl)pyrrolo[1,2-a]pyrimid-7-one

To 6-ethoxycarbonyl-2-(tert-butyl)-4*H*-pyrrolo[1,2-a]pyrimid-7-one (0.5 g, 1.9 mmol.) in DME (5 ml), tetrabutylammonium fluoride (4 ml, 4.0M in THF) was added, and a white foamy material formed. 2-Fluorobenzyl bromide (0.38 ml, 3 mmol.) was added and the mixture was stirred at room temperature for 2 days. Concentration of the reaction mixture *in vacuo* produced an oil, which was dissolved in acetone (20 ml) and diluted with water until the solution turned a slight cloudy. Partially concentration to remove acetone by nitrogen flow resulting precipitation. The precipitates were collected by filtration and washed with water (20 ml x2), ether (20 ml x3) and dried. The desired product (0.57g) was obtained with excellent purity; MS: 371 (M+H)$^+$, 325; NMR ($CDCl_3$, δ): 8.29 (1H, s), 7.43-7.14(5H, 2m), 5.99 (1H, s) 5.18 (2H,s), 4.35 (2H, q), 1.37(3H, s), 1.26 (9H, s).

Step 2D 6-Ethoxycarbonyl-3-(N-benzyl-N-methylaminomethyl)-2-(tert-butyl)-7-(2-fluorobenzyl)pyrrolo[1,2-a]pyrimid-7-one

Formaldehyde in water (1 drop) and N-benzyl-N-methylamine (2 drops) were added to acetic acid (1 ml) and stirred for 5 minutes. The 6-ethoxycarbonyl-2-(tert-butyl)-7-(2-fluorobenzyl)pyrrolo[1,2-a]pyrimid-7-one (20 mg, 0.05 mmol) was added. The solution was stirred at room temperature for 1 hour and concentrated to an oil. It was then neutralized by potassium carbonated (saturated) and the crude product was purified by prep TLC plate using $CHCl_3$/MEOH/$NH_4OH$ (400/50/2) to give the desired compound (11.1 mg); MS: 504(M+H)$^+$, 383. NMR ($CDCl_3$, δ): 8.18 (1H, s), 7.41-7.13 (9H, m), 5.93 (1H, s), 5.10(2H, s), 4.47 (2H, s), 4.36 (2H, q), 3.62 (2H, s), 2.08 (3H, s), 1.34 (9H, s). 1.34 (3H, t).

Following a procedure similar to that described above, the following intermediates were prepared:

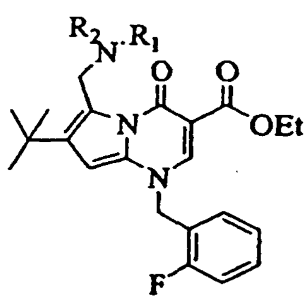

| Example | $R_1NR_2$ | MS (M+H$^+$) |
|---|---|---|
| 2A | BnNMe | 504 |
| 2B | 2-$PyCH_2CH_2NMe$ | 519 |

EXAMPLE 2.2

SYNTHESIS OF 1-(N-BENZYL-N-METHYLAMINOMETHYL)-2-(TERT-BUTYL)-4-(2-FLUOROBENZYL)-6-[(3-PHENYLPROPYLAMINO)CARBONYL]PYRROLO[1,2-A]PYRIMID-7-ONE

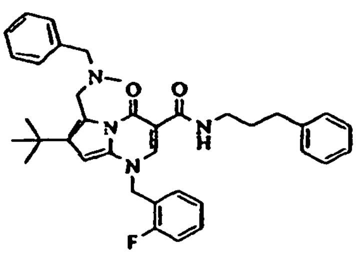

Step 2E 6-(3-Phenylpropylaminocarbonyl)-2-(tert-butyl)-7-(2-fluorobenzyl)pyrrolo[1,2-a]pyrimid-7-one

To a solution of 3-phenyl-l-propylamine (0.27 g, 2.0 mmol.) in DME (3 ml) under nitrogen atmosphere, triethy) aluminum (0.5 ml, 1.9 M in toluene) was added. The solution was stirred at room temperature for 0.5 hour, followed by addition of 6-ethoxycarbonyl-2-(tert-butyl)-7-(2-fluorobenzyl)pyrrolo[1,2-a]pyrimid-7-one (135mg, 0.5 mmole) . The solution was then heated at 50°C overnight and poured into a 6N HCl solution (5 ml). The crude product was extracted out by ethyl acetate (50 ml). The organic layer was filtered through a silica pad (2 g) and concentrated to give the desired product (100 mg) which was pure based on TLC (hexanetethyl acetate=1/1) and used for the next step; MS: 460 $(M+H)^+$.

Step 2F 6-(3-Phenylpropylaminocarbonyl)-2-(tert-butyl)-3-(N-benzyl-N-methylaminomethyl)-7-(2-fluorobenzyl)pyrrolo[1,2-a]pyrimid-7-one

To a solution of formaldehyde (37% aqueous, 1 drop) and N-benzyl-N-methylamine (1 drop) in acetic acid (1 ml) was added 6-(3-phenylpropylaminocarbonyl)-2-(tert-butyl)-4-(2-fluorobenzyl)pyrrolo[1,2-a]pyrimid-7-one (14mg, 0.03 mmol). The mixture was stirred at room temperature for 1 hour and concentrated in vacuo. The crude product was purified on prep-TLC plates using $CHCl_3$/MEOH/$NH_4OH$ (400/50/2) to give the desired product (13 mg); MS: 593 $(M+H)^+$, 472; NMR ($CDCl_3$, δ) 9.29 (1H, t), 8.43 (1H, s), 7.34-7.11 (14H, m), 5.95 (1H, s), 5.13 (2H, s), 4.42 (2H, s), 3.60 (2H, s), 3.46 (2H, m), 2.73 (2H, t), 2.23 (3H, s), 2.01 (2H, m), 1.38 (9H, s).

Following a procedure similar to that described above, the following compounds were prepared:

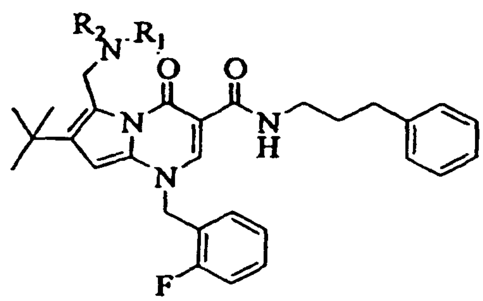

| Example | $R_1NR_2$ | MS $(M+H)^+$ |
|---|---|---|
| 2C | BnNMe | 593 |
| 2D | 2-$PyCH_2CH_2NMe$ | 608 |

EXAMPLE 3

SYNTHESIS OF 2-(TERT-BUTYL)-3-[N-(2-FLUOROPHENYL)ETHYL)AMINOMETHYL-5-(3-METHOXYPHENYL)-6-METHYL-7-(2-FLUOROBENZYL)IMMIDAZOLO[1,2-A]PYRIMID-4-ONE

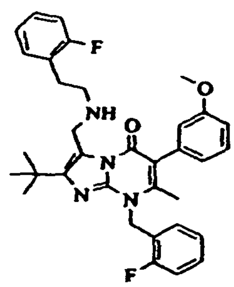

Step 3A. 2-(tert-Butyl)-3-formyl-5-(3-methoxyphenyl)-6-methyl-7-(2-fluorobenzyl)imidazolo[1,2-a]pyrimid-4-one

To a solution of 2-(tert-butyl)-5-(3-methoxyphenyl)-6-methyl-7-(2-fluorobenzyl)imidazolo[1,2-a]pyrimid-4-one (850 mg, 2.02 mmol.) in dry DMF (2 ml), $POCl_3$(1ml) was added . The mixture was heated at 50 °C for 10 minutes and ethyl acetate (200 ml) was added, followed by addition of saturated sodium bicarbonate slowly until it is neutral. The organic layer was dried and concentrated to give the crude product (910 mg) as a yellow solid; MS: 448 $(M+H)^+$.

Step 3B 2-(tert-Butyl)-3-[N-(2-fluorophenyl)ethyl]aminomethyl-5-(3-methoxyphenyl)-6-methyl-7-(2-fluorobenzyl) imidazolo[1,2-a]pyrimid-4-one

To a solution of 2-(tert-butyl)-3-formyl-5-(3-methoxyphenyl)-6-methyl-7-(2-fluorobenzyl)imidazolo[1,2-a]pyrimid-4-one (20 mg, 0.045 mmol.) in 1.2-dichloroethane (1 ml), 2-(2-fluorophenyl)-ethylamine (12.5 mg, 2.0 eq.) was added, followed by addition of sodium triacetoxyborohydride (48 mg, 5 eq.). The mixture was stirred at room temperature overnight. The product (12.9 mg) was isolated by a prep-TLC plate (0.5 mm thickness, 20x20 cm size) using $CHCl_3/MeOH/NH_4OH$ (400/50/2) as elution solvents; MS: 571$(M+H)^+$, 432. NMR ($CDCl_3$, δ) : 7.36-6.72 (12H, m), 5.63 (2H,s), 4.28(2H,s), 3.81 (3H,s), 3.05-2.87 (4H,m), 2.16(3H,s), 1.40(9H,s).

Following a procedure similar to that described above, the following compounds were prepared:

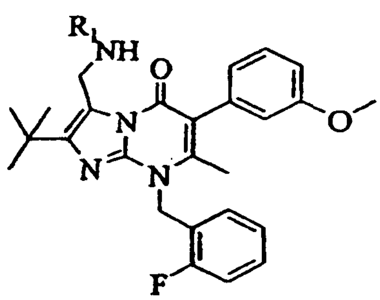

| Example | $R_1$ | MS $(M+H)^+$ |
|---|---|---|
| 3A | $(2\text{-}FPh)CH_2CH_2$ | 571 |
| 3B | $(2\text{-}Py)CH_2CH_2$ | 554 |
| 3C | $PhCH_2CH_2CH_2CH_2$ | 581 |
| 3D | $2\text{-}PyCH_2$ | 540 |
| 3E | $EtOCH_2CH_2CH_2$ | 535 |

EXAMPLE 4.1

SYNTHESIS OF 2-(1-METHOXYCARBONYL-1-METHYLETHYL)-3-{N-[(2-PYRIDYL)ETHYL]AMINOMETHYL}-5-BROMO-6-METHYL-7-(2-FLUOROBENZYL)IMMIDAZOLO[1,2-A]PYRIMID-4-ONE (INTERMEDIATE)

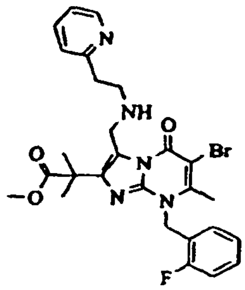

Step 4.1A <u>5-Bromo-6-methyl-2-(1-methoxycarbonyl-1-methylethyl)-7*H*-imidazolo[1,2-a]pyrimid-4-one</u>

Under nitrogen atmosphere, to a solution of 2-amino-5-bromo-4-hydroxy-6-methylpyrimidine (4.08 g, 20 mmol.) in dry DMF (80 ml), sodium hydride (800 mg, 20 mmol., 60% in mineral oil) was carefully added. The mixture was stirred at room temperature for 0.5 hour, followed by addition of methyl 4-bromo-2,2-dimethyl acetoacetate (20 mmol.). The mixture was then stirred at room temperature overnight. The resultant mixture was concentrated in vacuo, and the residue was dissolved in acetone (50 ml) and diluted with water (100 ml). Slowly concentration under reduced pressure resulted a precipitation. The solid was collected by filtration, washing with water, ether, and dried to yield the desired product; MS: 328 (M+H); proton NMR ($CDCl_3$): 1.64 (s, 6H), 2.55 (s, 3H), 3.77 (s, 3H), 7.40 (s, 1H).

Step 4.2B <u>5-Bromo-6-methyl-2-(1-methoxycarbonyl-1-methylethyl)-7-(2-fluorophenylmethyl)-imidazolo[1,2-a]pyrimid-4-one</u>

To a solution of 5-bromo-6-methyl-2-(1-methoxycarbonyl-1-methylethyl)-7*H*-imidazolo[1,2-a]pyrimid-4-one (2.4 mmol.) in DME (5 ml), tetrabutylammonium fluoride (4 ml, 1.0 M in THF) was added and followed by addition of 2-fluorobenzyl bromide (0.45 ml, 1.5 eq.). The mixture was stirred at room temperature overnight and then concentrated and purified by silica gel chromatography (hexane/ethyl acetate) to give the desired product as a white powder, MS: 436 (M+H); NMR ($CDCl_3$, δ): 1.59 (s, 6H), 2.60 (s, 3H), 3.64 (s, 3H), 5.68 (s, 2H), 7.00-7.38 (m, 4H), 7.51 (s, 1H).

Step 4.1C <u>3-{N-[2-(2-Pyridyl)ethyl]aminomethyl}-5-bromo-6-methyl-2-(1-methoxycarbonyl-1-methylethyl)-7-(2-fluorophenylmethyl)imidazolo[1,2-a]pyrimid-4-one</u>

To a solution of 2-(2-pyridyl)ethylamine (12 mg, 0.1 mmol.) and aqueous formaldehyde (0.01 ml) in acetic acid (1 ml), 5-bromo-6-methyl-2-(1-methoxycarbonyl-1-methylethyl)-7-(2-fluorophenylmethyl)-imidazolo[1,2-a]pyrimid-4-one (22 mg, 0.05 mmol.) was added. The solution was stirred at room temperature for 1 hours and directly purified by prep HPLC to give the product; MS :570 (M+H).

EXAMPLE 4.2

SYNTHESIS OF 2-(1-METHOXYCARBONYL-1-METHYLETHYL)-3-(N-METHYL-N-BENZYLAMINOMETHYL)-5-(3-METHOXYPHENYL)-6-METHYL-7-(2-FLUOROBENZYL)IMIDAZOLO[1,2-A]PYRIMID-4-ONE

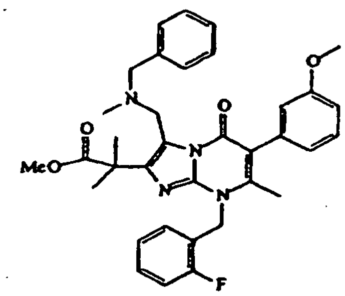

Step 4.2A 2-(1-Methoxycarbonyl-1-methylethyl)-5-(3-methoxyphenyl)-6-methyl-7-(2-fluorobenzyl)imidazolo[1,2-a]pyrimid-4-one

Into a mixture, of 2-(1-methoxycarbonyl-1-methylethyl)-5-(bromo)-6-methyl-7-(2-fluorobenzyl)imidazolo[1,2-a]pyrimid-4-one (200 mg, 458 μmol), 3-methoxyphenylboronic acid (139 mg, 916 μmol), potassium carbonate (190 mg, 1.4 mmol) in toluene (4 ml) and $H_2O$ (2 ml) were added under $N_2$ tetrakis(triphenylphosphine)palladium(0) (26 mg, 23 μmol). The resulting solution was stirred and refluxed in a ChemGlass pressure tube under $N_2$ for 2.5 hours. The solution was extracted with EtOAc and purified using Flash silica chromatography (hexane to hexane/EtOAc, 7/3) to give the desired product as an ecru solid in 83% yield; MS: 464 (M+H).

Step 4.2B 2-(1-Methoxycarbonyl-1-methylethyl)-3-(N-methyl-N-benzylaminomethyl)-5-(3-methoxyphenyl)-6-methyl-7-(2-fluorobenzyl)imidazolo[1,2-a]pyrimid-4-one

2-(1-Methoxycarbonyl-1-methylethyl)-5-(3-methoxyphenyl)-6-methyl-7-(2-fluorobenzyl)imidazolo[1,2-a]pyrimid-4-one (17.7 mg, 38 μmol) was added to a stirring solution of aqueous formaldehyde (one drop) and N-methylbenzylamine (0.1ml, 775 μmol) in acetic acid (1 ml), and then stirred at room temperature overnight. The solution was dried under $N_2$, made basic with $NaHCO_3$, extracted with dichloromethane and purified using prep silica TLC (hexane/EtOAc, 6/4) to give the product as an oil in 94% yield. $H^1$-NMR ($CDCl_3$): 1.69(s,6H), 2.07(s,3H), 2.16(s,3H), 3,58(s,2H), 3.61(s,3H), 3.81 (s,3H), 4.26(s,2H), 5.61(s,2H), 6.75(m,13H).

Following a procedure similar to that described above, the following compounds were prepared:

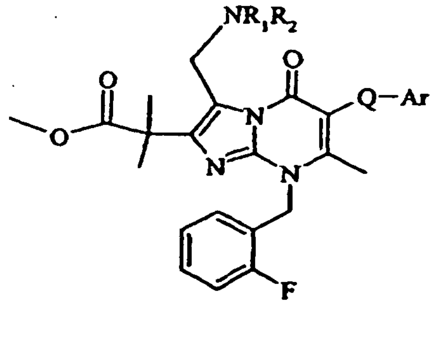

| Example | $R_1NR_2$ | Q-Ar | MS $(M+H)^+$ |
|---|---|---|---|
| 4A | BnNMe | 3-OMe-Ph | 597 |
| 4B | 2-$PyCH_2CH_2NMe$ | 3-OMe-Ph | 611 |
| 4C | $PhCH_2CH_2NMe$ | 3-OMe-Ph | 612 |
| 4D | 2-$FuranCH_2NMe$ | 3-OMe-Ph | 587 |
| 4E | 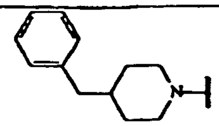 | 3-OMe-Ph | 651 |
| 4F | 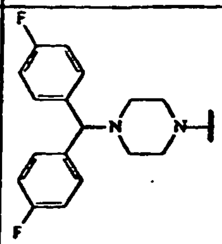 | 3-OMe-Ph | 763 |
| 4G | 2-$PyCH_2NH$ | 3-OMe-Ph | 584 |
| 4H | 2-$PyCH_2CH_2NMe$ | 2-F-3-OMePh | 630 |

EXAMPLE 5

SYNTHESIS OF 2-ETHOXYCARBONYLMETHYL-3-{N-METHYL-N-[2-(2-PYRIDYL)ETHYL]AMINOMETHYL}-5-(3-METHOXYPHENYL)-6-METHYL-7-(2-FLUOROBENZYL)IMIDAZOLO[1,2-A]PYRIMID-4-ONE

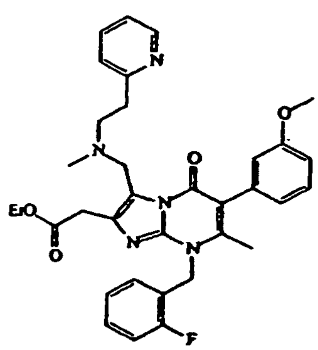

**Step 5A** 2-Ethoxycarbonylmethyl-5-bromo-6-methyl-7H-imidazolo[1,2-a]pyrimid-4-one.

A solution of 2-amino-5-bromo-6-methylpyrimid-4-one (8.16 g, 40 mmol.) in DMF (30 ml) was cooled down with ice-water bath. NaH (60% in oil, 1.76 g, 44 mmol.) was added in portions and the reaction mixture stirred at 0 °C for 30 min and then warmed to ambient temperature for 1h. Ethyl 4-chloroacetoacetate (6.91 g, 42 mmol) in 50 ml DMF was added dropwise in 3 h and the reaction mixture was stirred at ambient temperature overnight. Reaction mixture was quenched with saturated $NH_4Cl/H_2O$, and precipitates were filtered. The filtrate was concentrated *in vacuo* and the residue was partitioned between water and dichloromethane. The aqueous phase was extracted with dichloromethane (3 x 100 ml). The organic phases were combined, dried over sodium sulfate and concentrated to give the tilted compound (3.05g, 24%); MS 314 $(M+10^+$.

Step 5B 2-Ethoxycarbonylmethyl-5-bromo-6-methyl-7-(2-fluorobenzyl)-imidazolo[1,2-a]pyrimid-4-one.

A solution of 2-ethoxycarbonylmethyl-5-bromo-6-methyl-7H-imidazolo[1,2-a]pyrimid-4-one. (3.00 g, 9.55 mmol.) in DME (35 ml) was treated with 1M TBAF/THF (14.3 mL, 14.3 mmol.) and stirred at ambient temperature for 30 min. 2-Fluorobenzyl bromide (2.71 g, 14.3 mmol.) was introduced. The reaction mixture was stirred at ambient temperature overnight, concentrated *in vacuo*, and the residue was purified by flash chromatography (silica, 40% EtOAc/hexanes) to give the designed compound (356 mg, 9%). MS 424 $(M+H)^+$.

Step 5C 2-Ethoxycarbonylmethyl-5-(3-methoxyphenyl)-6-methyl-7-(2-fluorobenzyl)imidazolo[1,2-a]pyrimid-4-one.

2-Ethoxycarbonylmethyl-5-bromo-6-methyl-7-(2-fluorobenzyl)imidazolo[1,2-a]pyrimid-4-one (190 mg, 0.45 mmol.) in benzene/water (8 ml/5 ml) was added $K_2CO_3$ (155 mg, 1.12 mmol.), 3-methoxyphenylboronic acid (86 mg, 0.56 mmol.), and tetrakis(triphenylphosphine)palladium(0) (52 mg, 0.045 mmol.). The reaction mixture was deoxygenated with $N_2$ and heated at 90 °C for 16 h. The reaction mixture was partitioned between brine and EtOAc. The organic layer was dried (sodium sulfate), evaporated, purified by flash chromatography (silica, 45% EtOAc/hexane) to give the title compound. (93 mg, 46%); MS 450 $(M+H)^+$.

Step 5D 2-Ethoxycarbonylmethyl-3-{N-methyl-N-[2-(2-pyridyl)ethyl]aminomethyl}-5-(3-methoxyphenyl)-6-methyl-7-(2-fluorobenzyl)imidazolo[1,2-a]pyrimid-4-one.

A mixture of aqueous formaldehyde (37%, 1 drop) and amine (1 drop) in acetic acid (1.5 ml) was added 2-ethoxycarbonylmethyl-5-(3-methoxyphenyl)-6-methyl-7-(2-fluorobenzyl)imidazolo[1,2-a]pyrimid-4-one (20 mg, 0.045 mmol.) and the reaction mixture was stirred at ambient temperature for 12 h. The solvent was evaporated and the residue was partitioned between dichloromethane and saturated $NaHCO_3$/water, the organic layer was dried (sodium sulfate), evaporated and purified by prep. TLC (silica, 5% MeOH/dichloromethane) to give the titled compound; MS 598 $(M+H)^+$.

Replacing 2-(2-pyridyl)ethyl at the $R_1$ position with benzyl, gave 2-ethoxycarbonylmethyl-3-{N-methyl-N-benzyl)-5-(3-methoxyphenyl)-6-methyl-7-(2-fluorobenzyl)imidazolo[1,2-a]pyrimid-4-one. MS 583 $(M+H)^+$; $H^1$-NMR ($CDCl_3$): 1.23 (t,3H), 2.15 (s,3H), 2,47 (s,3H), 3.79 (s,2H), 3.83 (2,2H), 3.87 (s,3H), 4.14 (q, 2H), 4.30 (s,2H), 5.62 (s, 2H), 6.78-7.65 (m,13H).

Following a procedure similar to that described above, the following compounds were prepared:

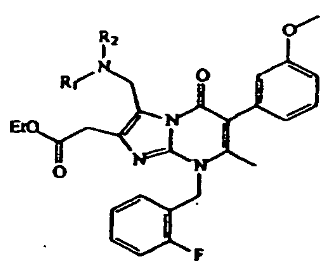

| Example | $R_1NR_2$ | MS (M+H) |
|---|---|---|
| 5A | 2-$PyCH_2CH_2NMe$ | 598 |
| 5B | BnNMe | 583 |
| 5C | BuNMe | 549 |
| 5D | 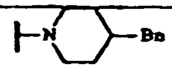 | 637 |
| 5E | 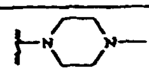 | 562 |

**Claims**

**1.** A compound having the following structure:

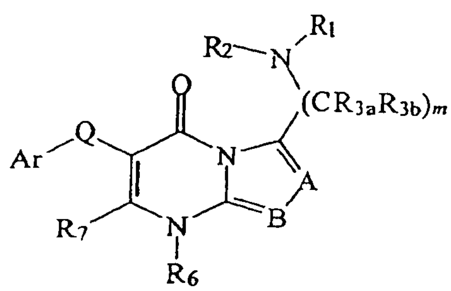

and stereoisomers, and pharmaceutically acceptable salts thereof, wherein:

A is independently selected from N or $CR_4$;
B is independently selected from N or $CR_5$;
Q is a direct bond or $-(CR_{8a}R_{8b})_r$-Z-$(CR_{10a}R_{10b})_s$-;
*m*, *r* and s are the same or different and selected from an integer from 0 to 6 ;
Z is a direct bond or -O-, -S-, $-NR_9-$, -SO-, $-SO_2-$, $-OSO_2-$, $-SO_2O-$, $-SO_2NR_9-$, $-NR_9SO_2-$, -CO-, -COO-, -OCO-, $-CONR_9-$, $-NR_9CO-$, $-NR_9CONR_{9a}$, $-OCONR_9-$ or $-NR_9COO-$;
$R_1$ is hydrogen, alkyl, substituted alkyl, aryl, substituted aryl, arylalkyl, substituted arylalkyl, heteroaryl, substituted heteroaryl, heteroarylalkyl, substituted heteroarylalkyl, - $C(R_{1a})(=NR_{1b})$, or $-C(NR_{1a}R_{1c})(=NR_{1b})$;
$R_2$ is hydrogen, alkyl or substituted alkyl;
or $R_1$ and $R_2$ taken together with the nitrogen atom to which they are attached form a heterocycle ring or a substituted heterocycle ring;
$R_{3a}$ and $R_{3b}$ are independently selected from hydrogen, alkyl, substituted alkyl, aryl, substituted aryl, arylalkyl, heterocycle, heterocyclealkyl, hydroxy, alkoxy, alkylthio, alkylamino, $CONR_{14}R_{15}$, or $-COOR_{14}$;
or $R_{3a}$ and $R_{3b}$ taken together with the carbon atom to which they are attached form a 3-6 membered homocyclic ring, substituted homocyclic ring, heterocyclic ring or substituted heterocyclic ring;
or $R_{3a}$ and $R_{3b}$ taken together form $=NR_{3c}$;
$R_4$ is hydrogen, halogen, cyano, nitro, alkyl, substituted alkyl, arylalkyl, substituted arylalkyl, heterocyclealkyl, substituted heterocyclealkyl, $-COR_{11}$, $-COOR_{11}$,
$-CONR_{12}R_{13}$, $-OR_{11}$, $-OCOR_{11}$, $-OSO_2R_{11}$, $-SR_{11}$, $-SO_2R_{11}$, $-NR_{12}R_{13}$, $-NR_{11}COR_{12}$,

$-NR_{11}CONR_{12}R_{13}$, $-NR_{11}SO_2R_{12}$ or $-NR_{11}SO_2NR_{12}R_{13}$; or $R_4$ and $R_1$, together with the atoms to which they are attached, form a 5-7 member heterocyclic ring or substituted heterocyclic ring;
or $R_4$ and $R_{3a}$, together with the atoms to which they are attached, form a 5-7 membered homocyclic ring, substituted homocyclic ring, heterocyclic ring or substituted heterocyclic ring;
$R_5$ is hydrogen, halogen, lower alkyl, arylalkyl, alkoxy, alkylthio, alkylamino, cyano or nitro;
$R_6$ is hydrogen, alkyl, substituted alkyl, aryl, substituted aryl, arylalkyl, substituted arylalkyl, heteroaryl, substituted heteroaryl, heteroarylalkyl or substituted hetcroarylalkyl;
$R_7$ is is hydrogen, halogen, cyano, alkyl, substituted alkyl, alkoxy, alkylthio, alkylsulfonyl or alkylamino;
Ar is aryl, heteroaryl, substituted aryl or substituted heteroaryl; and
$R_{1a}$, $R_{1b}$, $R_{1c}$, $R_{3c}$, $R_{8a}$, $R_{8b}$, $R_9$, $R_{9a}$, $R_{10a}$, $R_{10b}$, $R_{11}$, $R_{12}$, $R_{13}$, $R_{14}$ and $R_{15}$ are the same or different and at each occurrence independently hydrogen, acyl, alkyl, substituted alkyl, aryl, substituted aryl, arylalkyl, substituted arylalkyl, heterocycle, substituted heterocycle, heterocyclealkyl or substituted heterocyclealkyl;
or $R_{1a}$ and $R_{1b}$, $R_{8a}$ and $R_{8b}$, $R_{10a}$ and $R_{10b}$, or $R_{12}$ and $R_{13}$ taken together with the atom or atoms to which they are attached form a homocyclic ring, substituted homocyclic ring, heterocyclic ring or substituted heterocyclic ring.

**2.** The compound of claim 1 wherein Q is a direct bond and having the following structure:

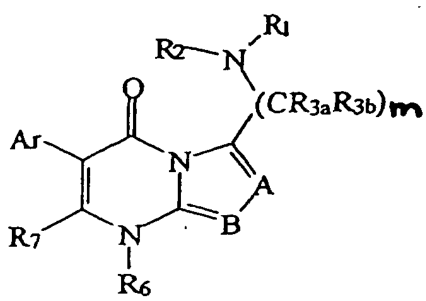

**3.** The compounds of claim 1 wherein Q is $-(CR_{8a}R_{8b})_r-Z-(CR_{10a}R_{10b})_s-$.

**4.** The compound of claim 3 wherein r is zero.

**5.** The compound of claim 3 wherein s is zero.

**6.** The compound of claim 3 wherein Z is carbonyl.

**7.** The compound of claim 2 wherein A is $CR_4$, B is nitrogen and having the following structure:

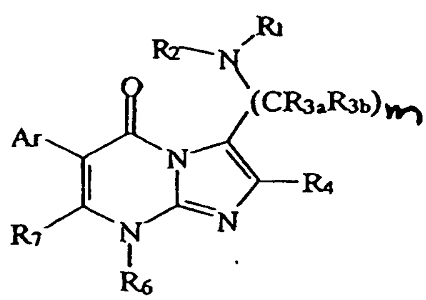

**8.** The compound of claim 2 wherein A is $CR_4$, B is $CR_5$ and having the following structure:

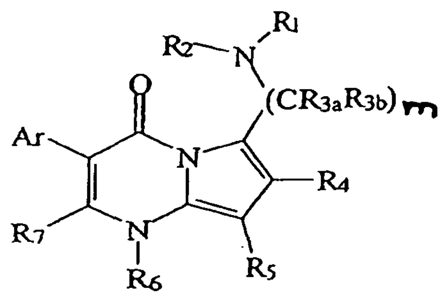

9. The compound of claim 2 wherein A is N, B is $CR_5$ and having the following structure:

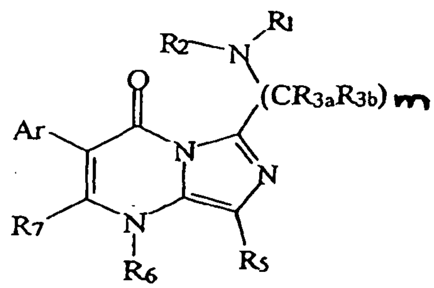

10. The compound of claim 2 wherein A is N, B is N and having the following structure:

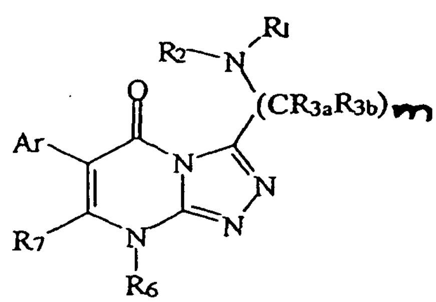

11. The compound of claim 1 wherein $R_6$ is arylalkyl or substituted arylalkyl.

12. The compound of claim 11 wherein $R_6$ is benzyl or substituted benzyl.

13. The compound of claim 1 wherein $R_7$ is alkyl.

14. The compound of claim 13 wherein $R_7$ is methyl.

15. The compound of claim 1 wherein $R_5$ is hydrogen.

16. The compound of claim 1 wherein $R_5$ is halogen, nitro or cyano.

17. The compound of claim 1 wherein $R_4$ is alkyl or substituted alkyl.

18. The compound of claim 1 wherein $R_{3a}$ and $R_{3b}$ are both hydrogen.

**19.** The compound of claim 1 wherein *m* is 1.

**20.** The compound of claim 1 wherein $R_2$ is alkyl.

**21.** The compound of claim 1 wherein $R_1$ is arylalkyl substituted arylalkyl, heteroarylalkyl or substituted heteroarylalkyl.

**22.** The compound of claim 21 wherein $R_1$ is benzyl or substituted benzyl.

**23.** The compound of claim 21 wherein $R_1$ is $-CH_2$(heteroaryl) or $-CH_2CH_2$(heteroaryl).

**24.** The compound of claim 1 wherein $R_1$ and $R_2$ taken together with the nitrogen atom to which they are attached form a heterocycle ring or substituted heterocycle ring.

**25.** The compound of claim 1 wherein the compound is:

3-(N-Benzyl-N-methyl)aminomethyl-2-(tert-butyl)-6-methyl-7-(2-fluorobenzyl)-5-(3-methoxyphenyl)imidazolo [1,2-a]pyrimid-4-one;
3-(N-(2-Pyridylmethyl))aminomethyl-2-(tert-butyl)-6-methyl-7-(2-fluorobenzyl)-5-(3-methoxyphenyl)imidazolo [1,2-a]pyrimid-4-one;
3-(N-(2-Pyridylmethyl)-N-methyl)aminomethyl-2-(tert-butyl)-6-methyl-7-(2-fluorobenzyl)-5-(3-methoxyphenyl)imidazolo[1,2-a]pyrimid-4-one;
3-[N-Methyl-N-(2-pyridylethyl)]aminomethyl-2-(tert-butyl)-6-methyl-7-(2-fluoro benzyl)-5-(3-methoxyphenyl) imidazolo[1,2-a]pyrimid-4-one;
3-[N-(2-Furanmethyl)-N-methyl]aminomethyl-2-(tert-butyn-6-methyl-7-(2-fluorobenzyl)-5-(3-methoxyphenyl) imidazolo[1,2-a]pyrimid-4-one;
3-[N-Methyl-N-(2-pyridylethyl)]aminomethyl-2-(ethoxycarbonylmethyl)-6-methyl-7-(2-fluorobenzyl)-5-(3-methoxyphenyl)imidazolo[1,2-a]pyrimid-4-one;
1-(N-Benzyl-N-methyl)aminomethyl-2-(tert-butyl)-4-(2-fluorobenzyl)-6-(3-phenylpropylaminocarbonyl)pyrrolo [1,2-a]pyrimid-7-one;
1-[(N-Methyl-N-(2-pyridylethyl)]aminomethyl-2-(tert-butyl)-4-(2-fluorobenzyl)-6-(3-phenylpropylaminocarbonyl)pyrrolo[1,2-a]pyrimid-7-one;
1-[(N-Methyl-N-(2-pyridylethyl)]aminomethyl-2-(tert-butyl)-4-(2-fluorobenzyl)-5-methyl-6-(3-methoxyphenyl) pyrrolo[1,2-a]pyrimid-7-one;
3-(N-Benzyl-N-methyl)aminomethyl-2-(t-butyl)-4-(2-fluorobenzyl)-5-methyl-6-(3-methoxyphenyl)imidazolo [3,4-a]pyrimid-7-one;
1-[N-Methyl-N-(2-pyridylethyl)]aminomethyl-4-(2-fluorobenzyl)-5-methyl-6-(3-methoxyphenyl)imidazolo [3,4-a]pyrimid-7-one;
1-[N-(2-Furanmethyl)-N-methyl]aminomethyl-4-(2-fluorobenzyl)-5-methyl-6-(3-methoxyphenyl)imidazolo [3,4-a]pyrimid-7-one;
3-(N-Benzyl-N-methyl)aminomethyl-6-methyl-7-(2-fluorobenzyl)-5-(3-methoxyphenyl)-1,2,4-triazolo[4,3-a] pyrimid-4-one;
3-[N-Methyl-N-(2-pyridylethyl)]aminomethyl-6-methyl-7-(2-fluorobenzyl)-5-(3-methoxyphenyl)-1,2,4-triazolo [4,3-a]pyrimid-4-one;
3-[N-(2-Furanmethyl)-N-methyl]aminomethyl-6-methyl-7-(2-fluorobenzyl)-5-(3-methoxyphenyl)-1,2,4-triazolo [4,3-a]pyrimid-4-one;
1-(N-Benzyl-N-methyl)aminomethyl-2-(1-methoxycarbonyl-1-methylethyl)-7-(2-fluorobenzyl)-6-methyl-5-(3-methoxyphenyl)imidazolo[1,2-a]pyrimid-4-one; or
1-[N-(2-Pyridylethyl)-N-methyl]aminomethyl-2-(1-methoxycarbonyl-1-methylethyl)-7-(2-fluorobenzyl)-6-methyl-5-(3-methoxyphenyl)imidazolo[1,2-a]pyrimid-4-one.

**26.** A pharmaceutical composition comprising a compound of claim 1 and a pharmaceutically acceptable carrier or diluent.

**27.** The compound of claim 1 for use in a method for antagonizing gonadotropin-releasing hormone in a subject in need thereof, the method comprising administering to the subject an effective amount of the compound.

**28.** The pharmaceutical composition of claim 26 for use in a method for treating a sex-hormone related condition of a subject in need thereof, comprising administering to the subject an effective amount of the pharmaceutical composition.

**29.** The pharmaceutical composition of claim 28 wherein the sex-hormone related condition is cancer, benign prostatic hypertrophy or myoma of the uterus.

**30.** The pharmaceutical composition of claim 29 wherein the cancer is prostatic cancer, uterine cancer, breast cancer or pituitary gonadotroph adenomas

**31.** The pharmaceutical composition of claim 28 wherein the sex-hormone related condition is endometriosis, polycystic ovarian disease, uterine fibroids or precocious puberty.

**32.** The pharmaceutical composition of claim 26 for use in a method for preventing pregnancy of a subject in need thereof, comprising administering an effective amount of the pharmaceutical composition.

**33.** The pharmaceutical composition of claim 26 for use in a method for treating lupus erythematosis, irritable bowel syndrome, premenstrual syndrome, hirsutism, short stature or sleep disorders of a subject in need thereof, comprising administering to the subject an effective amount of the pharmaceutical composition.

**Patentansprüche**

**1.** Verbindung mit der folgenden Struktur:

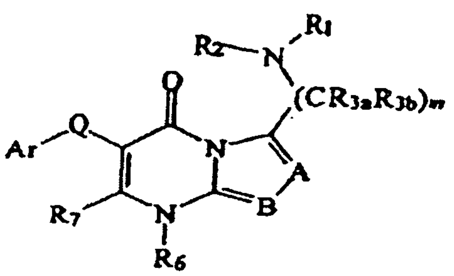

und Stereoisomere und pharmazeutisch annehmbare Salze davon, worin:

A unabhängig ausgewählt ist aus N oder $CR_4$;

B unabhängig ausgewählt ist aus N oder $CR_5$;

Q eine direkte Bindung oder $-(CR_{8a}R_{8b})_r$-Z-$(CR_{10a}R_{10b})_s$- ist;

*m*, *r* und *s* gleich oder voneinander verschieden sind und ausgewählt sind aus einer ganzen Zahl von 0 bis 6;

Z eine direkte Bindung oder -O-, -S-, $-NR_9-$, -SO-, $-SO_2-$, $-OSO_2-$, $-SO_2O-$, $-SO_2NR_9-$, $-NR_9SO_2-$, -CO-, -COO-, -OCO-, $-CONR_9-$, $-NR_9CO-$, $-NR_9CONR_{9a}$, $-OCONR_9-$ oder $-NR_9COO-$ ist;

$R_1$ Wasserstoff, Alkyl, substituiertes Alkyl, Aryl, substituiertes Aryl, Arylalkyl, substituiertes Arylalkyl, Heteroaryl, substituiertes Heteroaryl, Heteroarylalkyl, substituiertes Heteroarylalkyl, $-C(R_{1a})(=NR_{1b})$ oder $-C(NR_{1a}R_{1c})(=NR_{1b})$ ist;

$R_2$ Wasserstoff, Alkyl oder substituiertes Alkyl ist;

oder $R_1$ und $R_2$ zusammen mit dem Stickstoffatom, an welches sie gebunden sind, einen heterocyclischen Ring oder einen substituierten heterocyclischen Ring bilden;

$R_{3a}$ und $R_{3b}$ unabhängig ausgewählt sind aus Wasserstoff, Alkyl, substituiertem Alkyl, Aryl, substituiertem Aryl, Arylalkyl, einer heterocyclischen Gruppe, einer heterocyclischen Alkylgruppe, Hydroxy, Alkoxy, Alkylthio,

Alkylamino, $CONR_{14}R_{15}$ oder $-COOR_{14}$;

oder $R_{3a}$ und $R_{3b}$ zusammen mit dem Kohlenstoffatom, an welches sie gebunden sind, einen 3-6-gliedrigen homocyclischen Ring, substituierten homocyclischen Ring, heterocyclischen Ring oder substituierten heterocyclischen Ring bilden;

oder $R_{3a}$ und $R_{3b}$ zusammen $=NR_{3c}$ bilden;

$R_4$ Wasserstoff, Halogen, Cyano, Nitro, Alkyl, substituiertes Alkyl, Arylalkyl, substituiertes Arylalkyl, eine heterocyclische Alkylgruppe, eine substituierte heterocyclische Alkylgruppe, $-COR_{11}$, $-COOR_{11}$, $-CONR_{12}R_{13}$, $-OR_{11}$. $-OCOR_{11}$, $-OSO_2R_{11}$, $-SR_{11}$, $-SO_2R_{11}$, $-NR_{12}R_{13}$, $-NR_{11}COR_{12}$, $-NR_{11}CONR_{12}R_{13}$, $-NR_{11}SO_2R_{12}$ oder $-NR_{11}SO_2NR_{12}R_{13}$ ist; oder $R_4$ und $R_1$ zusammen mit den Atomen, an welche sie gebunden sind, einen 5-7-gliedrigen heterocyclischen Ring oder substituierten heterocyclischen Ring bilden;

oder $R_4$ und $R_{3a}$ zusammen mit den Atomen, an welche sie gebunden sind, einen 5-7-gliedrigen homocyclischen Ring, substituierten homocyclischen Ring, heterocyclischen Ring oder substituierten heterocyclischen Ring bilden;

$R_5$ Wasserstoff, Halogen, Niederalkyl, Arylalkyl, Alkoxy, Alkylthio, Alkylamino, Cyano oder Nitro ist;

$R_6$ Wasserstoff, Alkyl, substituiertes Alkyl, Aryl, substituiertes Aryl, Arylalkyl, substituiertes Arylalkyl, Heteroaryl, substituiertes Heteroaryl, Heteroarylalkyl oder substituiertes Heteroarylalkyl ist;

$R_7$ Wasserstoff, Halogen, Cyano, Alkyl, substituiertes Alkyl, Alkoxy, Alkylthio, Alkylsulfonyl oder Alkylamino ist;

Ar Aryl, Heteroaryl, substituiertes Aryl oder substituiertes Heteroaryl ist; und

$R_{1a}$, $R_{1b}$, $R_{1c}$, $R_{3c}$, $R_{8a}$, $R_{8b}$, $R_9$, $R_{9a}$, $R_{10a}$, $R_{10b}$, $R_{11}$, $R_{12}$, $R_{13}$, $R_{14}$ und $R_{15}$ gleich oder voneinander verschieden sind und bei jedem Vorkommen unabhängig voneinander Wasserstoff, Acyl, Alkyl, substituiertes Alkyl, Aryl, substituiertes Aryl, Arylalkyl, substituiertes Arylalkyl, eine heterocyclische Gruppe, eine substituierte heterocyclische Gruppe, eine heterocyclische Alkylgruppe oder substituierte heterocyclische Alkylgruppe bedeuten;

oder $R_{1a}$ und $R_{1b}$, $R_{8a}$ und $R_{8b}$, $R_{10a}$ und $R_{10b}$ oder $R_{12}$ und $R_{13}$ zusammen mit dem Atom oder den Atomen, an welches bzw. welche sie gebunden sind, einen homocyclischen Ring, substituierten homocyclischen Ring, heterocyclischen Ring oder substituierten heterocyclischen Ring bilden.

**2.** Verbindung nach Anspruch 1, worin Q eine direkte Bindung ist und welche die folgende Struktur aufweist:

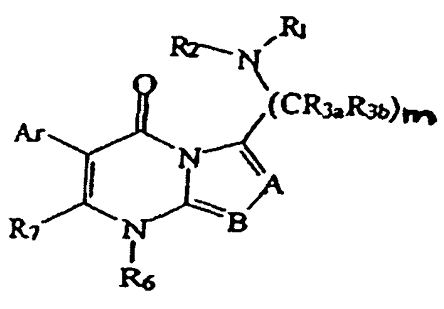

**3.** Verbindung nach Anspruch 1, worin Q $-(CR_{8a}R_{8b})_r-Z-(CR_{10a}R_{10b})_s-$ ist.

**4.** Verbindung nach Anspruch 3, worin r Null ist.

**5.** Verbindung nach Anspruch 3, worin s Null ist.

**6.** Verbindung nach Anspruch 3, worin Z Carbonyl ist.

**7.** Verbindung nach Anspruch 2, worin A $CR_4$ ist, B Stickstoff ist und welche die folgende Struktur aufweist:

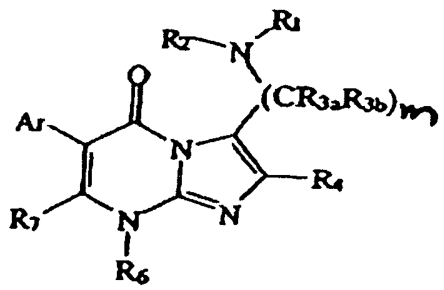

**8.** Verbindung nach Anspruch 2, worin A $CR_4$ ist, B $CR_5$ ist und welche die folgende Struktur aufweist:

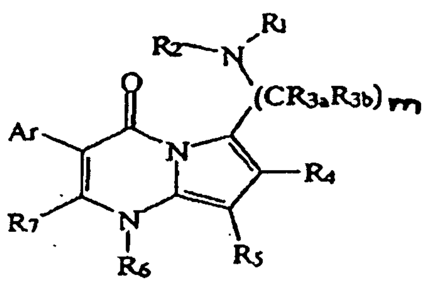

**9.** Verbindung nach Anspruch 2, worin A N ist, B $CR_5$ ist und welche die folgende Struktur aufweist:

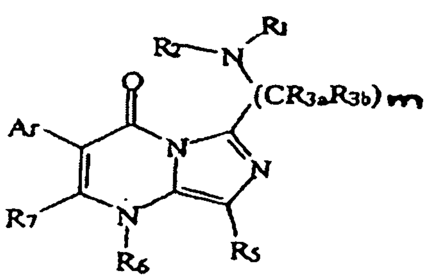

**10.** Verbindung nach Anspruch 2, worin A N ist, B N ist und welche die folgende Struktur aufweist:

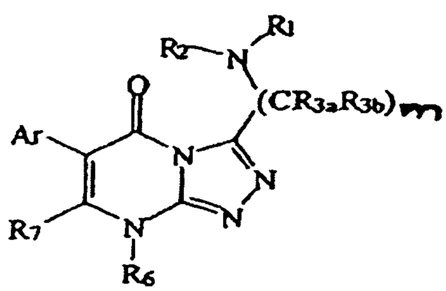

**11.** Verbindung nach Anspruch 1, worin $R_6$ Arylalkyl oder substituiertes Arylalkyl ist.

**12.** Verbindung nach Anspruch 11, worin $R_6$ Benzyl oder substituiertes Benzyl ist.

**13.** Verbindung nach Anspruch 1, worin $R_7$ Alkyl ist.

**14.** Verbindung nach Anspruch 13, worin $R_7$ Methyl ist.

**15.** Verbindung nach Anspruch 1, worin $R_5$ Wasserstoff ist.

**16.** Verbindung nach Anspruch 1, worin $R_5$ Halogen, Nitro oder Cyano ist.

**17.** Verbindung nach Anspruch 1, worin $R_4$ Alkyl oder substituiertes Alkyl ist.

**18.** Verbindung nach Anspruch 1, worin $R_{3a}$ und $R_{3b}$ beide Wasserstoff sind.

**19.** Verbindung nach Anspruch 1, worin *m* 1 ist.

**20.** Verbindung nach Anspruch 1, worin $R_2$ Alkyl ist.

**21.** Verbindung nach Anspruch 1, worin $R_1$ Arylalkyl, substituiertes Arylalkyl, Heteroarylalkyl oder substituiertes Heteroarylalkyl ist.

**22.** Verbindung nach Anspruch 21, worin $R_1$ Benzyl oder substituiertes Benzyl ist.

**23.** Verbindung nach Anspruch 21, worin $R_1$ $-CH_2$(Heteroaryl) oder $-CH_2CH_2$(Heteroaryl) ist.

**24.** Verbindung nach Anspruch 1, worin $R_1$ und $R_2$ zusammen mit dem Stickstoffatom, an welches sie gebunden sind, einen heterocyclischen Ring oder substituierten heterocyclischen Ring bilden.

**25.** Verbindung nach Anspruch 1, wobei die Verbindung:

3-(N-Benzyl-N-methyl)aminomethyl-2-(tert-butyl)-6-methyl-7-(2-fluorbenzyl)-5-(3-methoxyphenyl)imidazolo[1,2-a]pyrimid-4-on;
3-(N-(2-Pyridylmethyl))aminomethyl-2-(tert-butyl)-6-methyl-7-(2-fluorbenzyl)-5-(3-methoxyphenyl)imidazolo[1,2-a]pyrimid-4-on;
3-(N-(2-Pyridylmethyl)-N-methyl)aminomethyl-2-(tert-butyl)-6-methyl-7-(2-fluorbenzyl)-5-(3-methoxyphenyl)imidazolo[1,2-a]pyrimid-4-on;
3-[N-Methyl-N-(2-pyridylethyl)]aminomethyl-2-(tert-butyl)-6-methyl-7-(2-fluorbenzyl)-5-(3-methoxyphenyl)imidazolo[1,2-a]pyrimid-4-on;
3-[N-(2-Furanmethyl)-N-methyl]aminomethyl-2-(tert-butyl)-6-methyl-7-(2-fluorbenzyl)-5-(3-methoxyphenyl)imidazolo[1,2-a]pyrimid-4-on;
3-[N-Methyl-N-(2-pyridylethyl)]aminomethyl-2-(ethoxycarbonylmethyl)-6-methyl-7-(2-fluorbenzyl)-5-(3-methoxyphenyl)imidazolo[1,2-a]pyrimid-4-on;
1-(N-Benzyl-N-methyl)aminomethyl-2-(tert-butyl)-4-(2-fluorbenzyl)-6-(3-phenylpropylaminocarbonyl)pyrrolo[1,2-a]pyrimid-7-on;
1-[(N-Methyl-N-(2-pyridylethyl)]aminomethyl-2-(tert-butyl)-4-(2-fluorbenzyl)-6-(3-phenylpropylaminocarbonyl)pyrrolo[1,2-a]pyrimid-7-on;
1-[(N-Methyl-N-(2-pyridylethyl)]aminomethyl-2-(tert-butyl)-4-(2-fluorbenzyl)-5-methyl-6-(3-methoxyphenyl)pyrrolo[1,2-a]pyrimid-7-on;
3-(N-Benzyl-N-methyl)aminomethyl-2-(t-butyl)-4-(2-fluorbenzyl)-5-methyl-6-(3-methoxyphenyl)imidazolo[3,4-a]pyrimid-7-on;
1-[N-Methyl-N-(2-pyridylethyl)]aminomethyl-4-(2-fluorbenzyl)-5-methyl-6-(3-methoxyphenyl)imidazolo[3,4-a]pyrimid-7-on;
1-[N-(2-Furanmethyl)-N-methyl]aminomethyl-4-(2-fluorbenzyl)-5-methyl-6-(3-methoxyphenyl)imidazolo[3,4-a]pyrimid-7-on;
3-(N-Benzyl-N-methyl)aminomethyl-6-methyl-7-(2-fluorbenzyl)-5-(3-methoxyphenyl)-1,2,4-triazolo[4,3-a]pyrimid-4-on;

3-[N-Methyl-N-(2-pyridylethyl)]aminomethyl-6-methyl-7-(2-fluorbenzyl)-5-(3-methoxyphenyl)-1,2,4-triazolo [4,3-a]pyrimid-4-on;
3-[N-(2-Furanmethyl)-N-methyl]aminomethyl-6-methyl-7-(2-fluorbenzyl)-5-(3-methoxyphenyl)-1,2,4-triazolo [4,3-a]pyrimid-4-on;
1-(N-Benzyl-N-methyl)aminomethyl-2-(1-methoxycarbonyl-1-methylethyl)-7-(2-fluorbenzyl)-6-methyl-5-(3-methoxyphenyl)imidazolo[1,2-a]pyrimid-4-on; oder
1-[N-(2-Pyridylethyl)-N-methyl]aminomethyl-2-(1-methoxycarbonyl-1-methylethyl)-7-(2-fluorbenzyl)-6-methyl-5-(3-methoxyphenyl)imidazolo[1,2-a]pyrimid-4-on ist.

**26.** Pharmazeutische Zusammensetzung, umfassend eine Verbindung nach Anspruch 1 und einen pharmazeutisch annehmbaren Träger oder ein pharmazeutisch annehmbares Verdünnungsmittel.

**27.** Verbindung nach Anspruch 1 zur Verwendung in einem Verfahren zum Antagonisieren des Gonadotropin-Releasing-Hormons in einem Subjekt, welches dieses benötigt, wobei das Verfahren das Verabreichen einer wirksamen Menge der Verbindung an das Subjekt umfasst.

**28.** Pharmazeutische Zusammensetzung nach Anspruch 26 zur Verwendung in einem Verfahren zum Behandeln eines mit einem Sexualhormon zusammenhängenden Zustands bzw. Leidens eines Subjekts, welches dieses benötigt, umfassend das Verabreichen einer wirksamen Menge der pharmazeutischen Zusammensetzung an das Subjekt.

**29.** Pharmazeutische Zusammensetzung nach Anspruch 28, wobei es sich bei dem mit einem Sexualhormon zusammenhängenden Zustand bzw. Leiden um Krebs, benigne Prostatahypertrophie oder ein Uterusmyom handelt.

**30.** Pharmazeutische Zusammensetzung nach Anspruch 29, wobei es sich bei dem Krebs um Prostatakrebs, Gebärmutterkrebs, Brustkrebs oder gonadotrope Hypophysenadenome handelt.

**31.** Pharmazeutische Zusammensetzung nach Anspruch 28, wobei es sich bei dem mit einem Sexualhormon zusammenhängenden Zustand bzw. Leiden um Endometriose, das Syndrom der polyzystischen Ovarien, Uterusfibroide oder vorzeitige Pubertät handelt.

**32.** Pharmazeutische Zusammensetzung nach Anspruch 26 zur Verwendung in einem Verfahren zum Verhüten einer Schwangerschaft eines Subjekts, welches dieses benötigt, umfassend das Verabreichen einer wirksamen Menge der pharmazeutischen Zusammensetzung.

**33.** Pharmazeutische Zusammensetzung nach Anspruch 26 zur Verwendung in einem Verfahren zum Behandeln von Lupus erythematodes, Reizkolon, prämenstruellem Syndrom, Hirsutismus, Kleinwuchs oder Schlafstörungen eines Subjekts, welches dieses benötigt, umfassend das Verabreichen einer wirksamen Menge der pharmazeutischen Zusammensetzung an das Subjekt.

## Revendications

**1.** Un composé possédant la structure suivante :

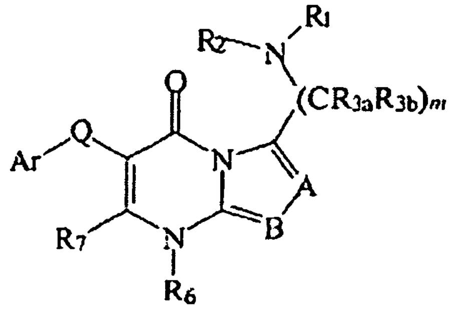

et les stéréo-isomères, et des sels de celui-ci acceptable sur le plan pharmaceutique, dans lequel :

A est choisi indépendamment de N ou $CR_4$ ;
B est choisi indépendamment de N ou $CR_5$ ;
Q est un lien direct ou $-(CR_{8a}R_{8b})_r$-Z-$(CR_{10a}R_{10b})_s$- ;
*m, r* et *s* sont identiques ou différents et choisis depuis un nombre entier de 0 à 6 ;
Z est un lien directe ou -O- , -S-, $-NR_9-$, -SO-, $-SO_2-$, $-OSO_2-$, $-SO_2O-$, $-SO_2NR_9-$, $-NR_9SO_2-$, -CO-, -COO-, -OCO-, $-CONR_9-$, $-NR_9CO-$, $-NR_9CONR_{9a}-$, $-OCONR_9-$ ou - $NR_9COO-$ ;
$R_1$ est un hydrogène, un alkyle, un alkyle substitué, un aryle, un aryle substitué, un arylalkyle, un arylalkyle substitué, un hétéroaryle, un hétéroaryle substitué, un hétéroarylalkyle, un hétéroarylalkyle substitué, $C(R_{1a})(=NR_{1b})$, ou $-C(NR_{1a}R_{1c})(=NR_{1b})$ ;
$R_2$ est un hydrogène, un alkyle ou un alkyle substitué ;
ou $R_1$ et $R_2$ pris ensemble avec l'atome d'azote auquel ils sont attachés forment un anneau hétérocycle ou un anneau hétérocycle substitué ;
$R_{3a}$ et $R_{3b}$ sont choisis indépendamment entre un hydrogène, un alkyle, un alkyle substitué, un aryle, un aryle substitué, un arylalkyle, une hétérocycle, un hétérocyclealkyle, un hydroxy, un alkoxy, un alkylthio, un alkylamino, un $CONR_{14}R_{15}$ ou $-COOR_{14}$ ;
ou $R_{3a}$ et $R_{3b}$ pris ensemble avec l'atome de carbone auquel ils sont attachés forment un anneau homocylique de 3-6 membres, un anneau homoclyclique substitués, un anneau hétérocyclique ou un anneau hétérocyclique substitué ;
ou $R_{3a}$ et $R_{3b}$ pris ensemble forment $=NR_{3c}$ ;
$R_4$ est un hydrogène, un halogène, un cyano, un nitro, un alkyle, un alkyle substitué, un arylalkyle, un arylalkyle substitué, un hétérocyclealkyle, un hétérocyclealkyle substitué, $-COR_{11}$, $-COOR_{11}$, $-CONR_{12}R_{13}$, $-OR_{11}$, $-OCOR_{11}$, - $OSO_2R_{11}$, $-SR_{11}$, $-SO_2R_{11}$, $-NR_{12}R_{13}$, $-NR_{11}COR_{12}$, $-NR_{11}CONR_{12}R_{13}$, $-NR_{11}SO_2R_{12}$ ou - $NR_{11}SO_2NR_{12}R_{13}$; ou $R_4$ et $R_1$, pris ensemble avec les atomes auxquels ils sont attachés, forment un anneau hétérocyclique de 5-7 membres ou un anneau hétérocyclique substitué ;
ou $R_4$ et $R_{3a}$ ensemble avec les atomes auxquels ils sont attachés, forment un anneau homocylique de 5-7 membres, un anneau homoclyclique substitués, un anneau hétérocyclique ou un anneau hétérocyclique substitué ;
$R_5$ est un hydrogène, un halogène, un alkyle inférieur, un arylalkyle, un alkoxy, un akylthio, un alkylamino, un cyano ou nitro ;
$R_6$ est un hydrogène, un alkyle, un alkyle substitué, un aryle, un aryle substitué, un arylalkyle, un arylalkyle substitué, un hétéroalkyle, un hétéroalkyle substitué, un hétéroarylalkyle ou un hétéroarylalkyle substitué ;
$R_7$ est un hydrogène, un halogène, un cyno, un alkyle, un alkyle substitué, un alkoxy, un alkylthio, alkylsulfonyle ou un alkylamino ;
Ar est un aryle, un hétéroaryle, un aryle substitué ou un hétéroaryle substitué ; et
$R_{1a}$, $R_{1b}$, $R_{1c}$, $R_{3c}$, $R_{8a}$, $R_{8b}$, $R_9$, $R_{9a}$, $R_{10a}$, $R_{10b}$, $R_{11}$, $R_{12}$, $R_{13}$, $R_{14}$ et $R_{15}$ sont identiques ou différent et à chaque survenue indépendamment un hydrogène, un acyle, un alkyle, un alkyle substitué, un aryle, une aryle substitué, une arylalkyle, un arylalkyle substitué, un hétérocycle, un hétérocycle substitué, un hétérocyclealkyle ou un hétérocyclealkyle substitué ;
ou $R_{1a}$ et $R_{1b}$, $R_{8a}$ et $R_{8b}$, $R_{10a}$ et $R_{10b}$, ou $R_{12}$ et $R_{13}$ pris ensemble avec l'atome ou les atomes auxquels ils sont attachés forment un anneau homocyclique, un anneau homocyclique substitué, un anneau hétérocyclique ou un anneau hétérocyclique substitué.

**2.** Le composé selon la revendication 1 dans lequel Q est un lien direct et possède la structure suivante :

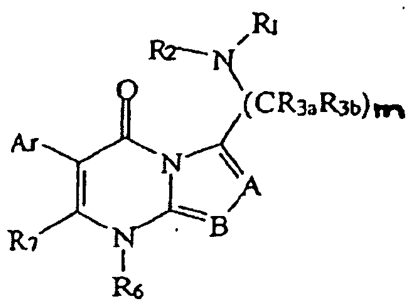

**3.** Le composé selon la revendication 1 dans lequel Q est $-(CR_{8a}R_{8b})_r-Z-(CR_{10a}R_{10b})_s-$.

**4.** Le composé selon la revendication 3 dans lequel r est zéro.

**5.** Le composé selon la revendication 3 dans lequel s est zéro.

**6.** Le composé selon la revendication 3 dans lequel Z est un carbonyle.

**7.** Le composé selon la revendication 2 dans lequel A est un $CR_4$, B est un azote et possède la structure suivante :

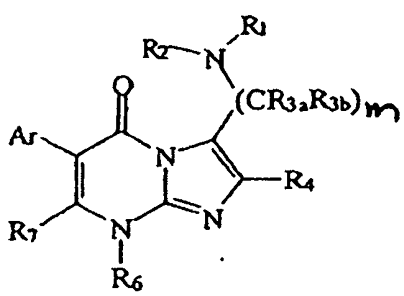

**8.** Le composé selon la revendication 2 dans lequel A est un $CR_4$, B est un $CR_5$ et possède la structure suivante :

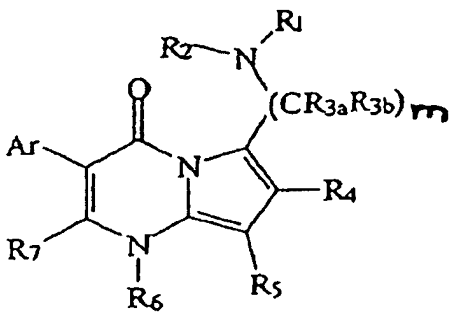

**9.** Le composé selon la revendication 2 dans lequel A est un N, B est un $CR_5$ et possède la structure suivante :

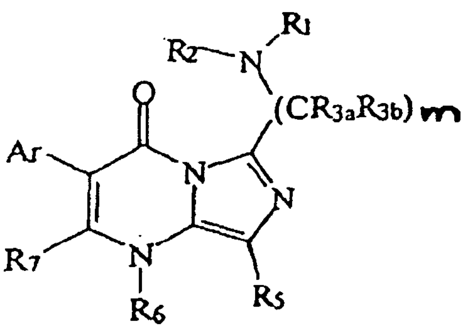

**10.** Le composé selon la revendication 2 dans lequel A est un N, B est un N et possède la structure suivante :

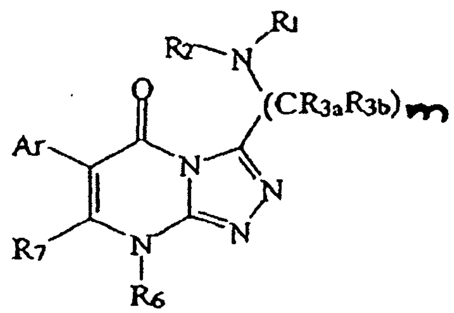

**11.** Le composé selon la revendication 1 dans lequel $R_6$ est un arylalkyle ou un arylalkyle substitué.

**12.** Le composé selon la revendication 11 dans lequel $R_6$ est un benzyle ou un benzyle substitué

**13.** Le composé selon la revendication 1 dans lequel $R_7$ est un alkyle.

**14.** Le composé selon la revendication 13 dans lequel $R_7$ est un méthyle.

**15.** Le composé selon la revendication 1 dans lequel $R_5$ est un hydrogène.

**16.** Le composé selon la revendication 1 dans lequel $R_5$ est un halogène, un nitro ou un cyno.

**17.** Le composé selon la revendication 1 dans lequel $R_4$ est un alkyle ou un alkyle substitué.

**18.** Le composé selon la revendication 1 dans lequel $R_{3a}$ et $R_{3b}$ sont tous le deux des hydrogènes.

**19.** Le composé selon la revendication 1 dans lequel *m* est 1.

**20.** Le composé selon la revendication 1 dans lequel $R_2$ est un alkyle.

**21.** Le composé selon la revendication 1 dans lequel $R_1$ est un arylalkyle, un arylalkyle substitué, un hétéroarylalkyle ou un hétéroarylalkyle substitué.

**22.** Le composé selon la revendication 21 dans lequel $R_1$ est un benzyle ou un benzyle substitué.

**23.** Le composé selon la revendication 21 dans lequel $R_1$ est un $-CH_2$(hétéroaryle) ou un $-CH_2CH_2$(hétéroaryle).

**24.** Le composé selon la revendication 1 dans lequel $R_1$ et $R_2$ pris ensemble avec l'atome d'azote auquel ils sont attachés, forment un anneau hétérocyle ou un anneau hétérocycle substitué.

**25.** Le composé selon la revendication 1 dans lequel le composé est :

3- (N-Benzyle-N-méthyle)aminométhyle-2- (tert-butyle)-6-méthyle-7-(2-fluorobenzyle)-5- (3-méthoxyphenyle) imidazolo[1,2-a]pyrimid-4-one;
3-(N-(2-Pyridylméthyle))aminométhyle-2-(tert-butyle)-6-méthyle-7-(2-fluorobenzyle)-5-(3-méthoxyphenyl) imidazolo [ 1, 2-a] pyrimid-4-one;
3-(N-(2-Pyridylméthyle)-N-méthyle)amionométhyle-2-(tert-butyle)-6-méthyle-7-(2fluorobenzyle)-5- (3-méthoxyphényle) imidazolo [ 1, 2-a] pyrimid-4-one;
3- [N-Méthyle-N- (2-pyridyléthyle)] aminométhyle-2- (tert-butyle)-6-méthyle-7- (2-fluorobenzyle)-5- (3-méthoxyphényl) imidazolo [ 1, 2-a] pyrimid-4-one;
3- [N- (2-Furanméthyle)-N-méthyle] aminométhyle-2- (tert-butyle)-6-méthyle-7- (2-fluorobenzyle)-5- (3-méthoxyphényle) imidazolo[1,2-a] pyrimid-4-one;
3- [N-Méthyle-N- (2-pyridyléthyle)] aminométhyle-2- (éthoxycarbonylméthyle)-6-méthyle-7- (2-fluorobenzyle)-5- (3-méthoxyphényle) imidazolo[ 1, 2-a] pyrimid-4-one;
1-(N-Benzyle-N-méthyle)aminométhyle-2-(tert-butyle)-4-(2-fluorobenzyle)-6-(3-phénylpropylaminocarbonyle) pyrrolo [ 1, 2-a] pyrimid-7-one;
1-[(N-Méthyle-N-(2-pyridyléthyle)]aminométhyle-2-(tert-butyle)-4-(fluorobenzyle)-6-(3-phénylpropylaminocarbonyle) pyrrolo[ 1,2-a] pyrimid-7-one;
1-[(N-Methyl-N-(2-pyridyléthyle)]aminométhyle-2-(tert-butyle)-4-(fluorobenzyle)-5-méthyle-6- (3-méthoxyphényle) pyrrolo [1, 2-a] pyrimid-7-one;
3- (N-Benzyle-N-méthyle) aminométhyle-2- (t-butyle)-4- (2-fluorobenzyle)-5-méthyle-6- (3-méthoxyphényle) imidazolo [3,4-a] pyrimid-7-one;
1-[N-Méthyle-N-(2-pyridyléthyle)]aminométhyle-4-(2-fluorobenzyle)-5-méthyle-6 (3-methoxyphényle) imidazolo[3,4-a]pyrimid-7-one ;
1-[N-(2-Furanméthyle)-N-méthyle] aminométhyle-4-(2-fluorobenzyle)-5-méthyle-6- (3-méthoxyphényle) imidazolo [3,4-a] pyrimid-7-one;
3- (N-Benzyle-N-méthyle) aminométhyle-6-méthyle-7- (2-fluorobenzyle)-5- (3-méthoxyphényl)-1,2,4-triazolo [4,3-a] pyrimid-4-one;
3-[N-Méthyle-N-(2-pyridyléthyle)] aminométhyle-6-méthyle-7- (2-fluorobenzyle)-5- (3-methoxyphenyl)-1,2,4-triazolo [4,3-a] pyrimid-4-one;
3- [N- (2-Furanméthyle)-N-méthyle]aminométhyle-6-méthyle-7-(2-fluorobenzyle)-5-(3-méthoxyphényle)-1,2,4-triazolo [4,3-a] pyrimid-4-one;
1- (N-Benzyle-N-méthyle) aminométhyle-2- ( 1-méthoxycarbonyle-1-méthyléthyle)-7-(2-fluorobenzyle)-6-méthyle-5-(3-méthoxyphényle) imidazolo[1,2-a] pyrimid-4-one; or
1-[N-(2-Pyridyléthyle)-N-méthyle]aminométhyle-2-(1-méthoxycarbonyle-1-méthyléthyle)-7- (2-fluorobenzyle)-6-méthyle-5- (3-méthoxyphényle)imidazolo[1,2-a]pyrimid-4one.

**26.** Une composition pharmaceutique comprenant un composé selon la revendication 1 et un transporteur ou un diluant acceptable sur le plan pharmaceutique.

**27.** Le composé selon la revendication 1 pour l'utilisation dans une méthode d'antagoniste de l'hormone gonadolibérine chez un sujet en ayant le besoin, la méthode comprenant l'administration à un sujet d'une quantité efficace du composé.

**28.** La composition pharmaceutique selon la revendication 26 pour l'utilisation dans une méthode pour le traitement d'une condition en relation avec une hormone sexuelle d'un sujet en ayant le besoin, comprenant l'administration au sujet d'une quantité efficace de la composition pharmaceutique.

**29.** La composition pharmaceutique selon la revendication 28 dans laquelle la condition en relation avec l'hormone sexuelle est un cancer, une hypertrophie bénigne de la prostate ou un myome utérin.

**30.** La composition pharmaceutique selon la revendication 29 dans laquelle le cancer est un cancer prostatique, un cancer utérin, un cancer mammaire ou un adénome gonadotrope pituitaire.

**31.** La composition pharmaceutique selon la revendication 28 dans laquelle la condition en relation avec l'hormone sexuelle est une endométriose, une maladie polykystique de l'ovaire, un fibrome utérin ou une puberté précoce.

**32.** La composition pharmaceutique selon la revendication 26 pour l'utilisation dans une méthode de prévention de la grossesse chez un sujet en ayant le besoin, comprenant l'administration d'une quantité efficace de la composition pharmaceutique.

**33.** La composition pharmaceutique selon la revendication 26 pour l'utilisation dans une méthode de traitement du lupus érythémateux, du syndrome du colon irritable, du syndrome prémenstruel, de l'hirsutisme, de la petite stature ou de désordre du sommeil chez des sujets en ayant le besoin, comprenant l'administration au sujet d'une quantité efficace de la composition pharmaceutique.